# EUROPÄISCHE PATENTSCHRIFT

(11) **EP 1 893 119 B1**
(45) Veröffentlichungstag und Bekanntmachung des Hinweises auf die Patenterteilung: **07.05.2014**
(21) Anmeldenummer: 06754209.2
(22) Anmeldetag: 08.06.2006
(51) Int. Cl.: A61B 19/00, A61B 17/86

(54) **CHIRURGISCHER SCHRAUBENBEHÄLTER**
SURGICAL SCREW CONTAINER
CONTENANT À VIS CHIRURGICAL

(30) Priorität: 22.06.2005 DE 102005030553
(43) Veröffentlichungstag der Anmeldung: 05.03.2008
(73) Patentinhaber: Aesculap AG, 78532 Tuttlingen (DE)
(72) Erfinder: NESPER, Markus, 78532 Tuttlingen (DE); WEISSHAUPT, Dieter, 78194 Immendingen (DE); PLEIL, Thomas, 78073 Bad Dürrheim (DE)
(74) Vertreter: Hoeger, Stellrecht & Partner Patentanwälte
(86) Internationale Anmeldenummer: PCT/EP2006/005458
(87) Internationale Veröffentlichungsnummer: WO 2006/136291

(56) Entgegenhaltungen:
- EP-A1- 1 258 437
- WO-A2-01/62136
- BE-A6- 1 005 149
- DE-U1- 20 318 732

## Beschreibung

Die vorliegende Erfindung betrifft einen chirurgischen Schraubenbehälter umfassend einen Aufnahmeteil und ein an diesem beweglich gelagertes Verschlußteil, wobei der Aufnahmeteil mindestens zwei Schraubenaufnahmen zum Aufnehmen von mindestens zwei Knochenschrauben aufweist, wobei das Verschlußteil relativ zum Aufnahmeteil in mindestens eine Verschlußstellung bringbar ist, in welcher keine der Schraubenaufnahmen zugänglich ist zum Einführen oder Entnehmen einer Knochenschraube, wobei das Verschlußteil von der Verschlußstellung in mindestens zwei Entnahmestellungen bringbar ist, in welchen jeweils nur eine einzige Schraubenaufnahme zugänglich ist zum Einführen oder Entnehmen einer einzelnen Knochenschraube.

In der Chirurgie werden zur Fixierung von Knochenfragmenten oder künstlichen Knochendeckeln im kranialen Bereich insbesondere kleine, flache Knochenplatten verwendet, die zum Beispiel mit selbstschneidenden und selbstbohrenden Schrauben am Knochen fixiert werden. Die hierfür verwendeten Knochenschrauben sind in der Regel sehr klein, so daß sie nur schwer handhabbar sind und leicht verloren gehen können. Daher werden sie zum Einsatz in der Chirurgie üblicherweise in einem chirurgischen Schraubenbehälter der eingangs beschriebenen Art bereitgestellt.

Bei den bekannten Schraubenbehältern hat es sich als nachteilig erwiesen, daß nach Entfernen des Verschlußteils alle Schraubenaufnahmen frei zugänglich sind. Dadurch kann zwar gleichzeitig auf alle im Schraubenbehälter befindlichen Schrauben zugegriffen werden, doch besteht auch die Gefahr, daß diese aus dem Schraubenbehälter herausfallen können, beispielsweise wenn der Schraubenbehälter umkippt. Da zudem bei einem chirurgischen Eingriff genau dokumentiert werden muß, welche Instrumente und gegebenenfalls auch welche Implantate verwendet wurden, muß eine verlorengegangene Knochenschraube so lange gesucht werden, bis sie wieder aufgefunden ist. Nur so kann vermieden werden, daß keine Instrumente oder Implantatteile in unbeabsichtigter Weise in einem Körper eines Patienten verbleiben.

Aus der WO 01/62136 A2 ist ein Schraubenbehälter der eingangs beschriebenen Art bekannt. Ein Magazin für chirurgische Befestigungsvorrichtungen ist in der EP 1 258 437 A1 beschrieben.

Es ist daher Aufgabe der vorliegenden Erfindung, einen chirurgischen Schraubenbehälter der eingangs beschriebenen Art so zu verbessern, daß keine Gefahr besteht, daß er sich in unbeabsichtigter Weise von einem Schraubenbehältermagazin lösen kann.

Diese Aufgabe wird bei einem chirurgischen Schraubenbehälter der eingangs beschriebenen Art erfindungsgemäß dadurch gelöst, daß am Schraubenbehälter mindestens ein Magazinkupplungsglied vorgesehen ist zum lösbaren Verbinden des Schraubenbehälters mit einem chirurgischen Schraubenbehältermagazin für mindestens einen chirurgischen Schraubenbehälter.

Der erfindungsgemäße chirurgische Schraubenbehälter stellt sicher, daß in jeder Entnahmestellung nur eine Schraubenaufnahme frei zugänglich ist. Damit die Schraube aus dem Schraubenbehälter entnommen werden kann, muß jedoch zunächst das Verschlußteil relativ zum Aufnahmeteil von der mindestens einen Verschlußstellung, d. h. von einer Verschlußstellung oder von mehreren Verschlußstellungen in eine oder mehrere Entnahmestellungen gebracht werden. Erst so ist ein Zugriff auf eine der mindestens zwei Schraubenaufnahmen möglich. Alle anderen Schraubenaufnahmen sind dabei jedoch nicht zugänglich. Dadurch kann keine, der in den anderen Schraubenaufnahmen befindlichen Schrauben aus dem Schraubenbehälter herausfallen. Ferner sei angemerkt, daß der chirurgische Schraubenbehälter auch zur Aufnahme von anderen Befestigungselementen oder Werkzeugen dienen kann, beispielsweise chirurgischen Nägeln oder Werkzeugeinsätzen, sogenannten "Bits". Grundsätzlich wäre es denkbar, den Schraubenbehälter für sich allein zu nutzen. Allerdings ist es auch denkbar, den Schraubenbehälter mit weiteren Schraubenbehältern für Schrauben unterschiedlicher Größen in einem gemeinsamen Schraubenmagazin anzuordnen. Um zu vermeiden, daß sich ein Schraubenbehälter in unerwünschter Weise vom Schraubenmagazin lösen kann, ist es günstig, daß am Schraubenbehälter mindestens ein Magazinkupplungsglied vorgesehen ist zum lösbaren Verbinden des Schraubenbehälters mit einem chirurgischen Schraubenbehältermagazin für mindestens einen chirurgischen Schraubenbehälter. Auf diese Weise kann ein Schraubenbehälter mit dem Schraubenbehältermagazin verbunden werden, ohne daß die Gefahr besteht, daß er sich in unbeabsichtigter Weise vom Schraubenbehältermagazin löst. Außerdem kann ein Schraubenbehälter auf einfache Weise ausgetauscht werden, beispielsweise dann, wenn für einen chirurgischen Eingriff größere oder andersartige Schrauben benötigt werden.

Der Aufbau und die Herstellung des Schraubenbehälters wird besonders einfach, wenn die Schraubenaufnahmen in Form von Steckbohrungen oder Sacklochbohrungen in einem Grundkörper des Aufnahmeteils ausgebildet sind. Die Schrauben können insbesondere mit einem mit einem Außengewinde versehenen Schraubenkörper voran in die Steckbohrungen oder Sacklochbohrungen eingesetzt sein, so daß ein Kopf der Schraube aus diesen herausragt.

Ein Übergang von der mindestens einen Verschlußstellung in die mindestens eine Entnahmestellung und umgekehrt wird besonders einfach, wenn das Verschlußteil am Aufnahmeteil um eine Drehachse verdrehbar gelagert ist.

Der Aufbau des Schraubenbehälters vereinfacht sich weiter, wenn Längsachsen der Schraubenaufnahmen parallel zur Drehachse verlaufen.

Ein individueller Zugriff auf eine einzige Schraubenaufnahme wird vereinfacht, wenn die mindestens zwei Schraubenaufnahmen konzentrisch um die Drehachse herum angeordnet sind.

Vorteilhafterweise sind die mindestens zwei Schraubenaufnahmen auf mindestens zwei konzentrischen Kreisen um die Drehachse herum angeordnet. Dadurch können deutlich mehr Schraubenaufnahmen am Schraubenbehälter vorgesehen werden als dies bei einer Anordnung der Schraubenaufnahmen auf nur einem zur Drehachse konzentrischen Kreis möglich ist.

Um das Verschlußteil sicher mit dem Aufnahmeteil verbinden und gegebenenfalls auch auf einfache Weise wieder von diesem lösen zu können, ist es günstig, wenn das Verschlußteil zum beweglichen Lagern am Aufnahmeteil mit diesem verrastbar ist. Eine Rastverbindung hat zudem den Vorteil, daß das Einnehmen einer Verbindungsstellung von Aufnahmeteil und Verschlußteil in der Regel für eine Bedienperson sowohl spürbar als auch hörbar ist.

Gemäß einer bevorzugten Ausführungsform der Erfindung kann vorgesehen sein, daß das Aufnahmeteil und das Verschlußteil zum beweglichen Lagern aneinander jeweils ein Kupplungselement tragen, die miteinander in Eingriff bringbar sind. Durch die beiden Kupplungselemente kann auf einfache Weise eine Verbindung zwischen dem Aufnahmeteil und dem Verschlußteil realisiert werden.

Um das Verschlußteil und das Aufnahmeteil in einer Kupplungsstellung sicher und auf einfache Weise aneinander und relativ zueinander bewegbar zu halten, ist es vorteilhaft, wenn die beiden Kupplungselemente in einer Kupplungsstellung relativ zueinander verdrehbar und mittels einer Rastverbindung aneinander gehalten sind.

Der Aufbau des Schraubenbehälters und seiner Teile wird weiter vereinfacht, wenn das eine der beiden Kupplungselemente ein Kupplungszapfen und wenn das andere Kupplungselement eine zum Kupplungszapfen korrespondierende Zapfenaufnahme ist.

Besonders günstig ist es, wenn der Kupplungszapfen die Drehachse definiert. Der Kupplungszapfen dient somit einerseits zum Verbinden des Aufnahmeteils mit dem Verschlußteil, zum anderen bildet er auch eine Lagerwelle.

Um auf einfache Weise eine Schraube aus dem Schraubenbehälter in einer der Entnahmestellungen entnehmen zu können, ist es besonders günstig, wenn das Verschlußteil mindestens eine Entnahmeöffnung aufweist und wenn die mindestens eine Entnahmeöffnung mindestens in einer der mindestens zwei Entnahmestellungen jeweils eine einzige Schraubenaufnahme freigibt. Beispielsweise können auch zwei oder noch mehr Entnahmeöffnungen vorgesehen sein, wobei jedoch in jeder Entnahmestellung jeweils nur eine einzige Schraubenaufnahme frei zugänglich ist.

Der Aufbau und die Herstellung des Verschlußteils vereinfacht sich, wenn es eine Deckplatte zum mindestens teilweisen Bedecken der Schraubenaufnahmen aufweist und wenn die mindestens eine Entnahmeöffnung in Form einer Durchbrechung der Deckplatte ausgebildet ist. Die Form der Durchbrechung kann insbesondere an die Form der Schraubenaufnahme angepaßt und/oder dieser ähnlich sein. Bei einem runden oder zylindrischen Schraubenbehälter kann die Deckplatte die Form einer flachen Scheibe aufweisen.

Besonders einfach wird die Herstellung des Verschlußteils, wenn die Durchbrechung eine Bohrung ist.

Eine Vielzahl von Entnahmestellungen kann auf besonders einfache Weise eingenommen werden, wenn die mindestens eine Entnahmeöffnung konzentrisch um die Drehachse angeordnetist. Zwischen einer und mehreren Entnahmestellungen und/oder Verschlußstellungen kann so einfach durch Verdrehen des Verschlußteils relativ zum Aufnahmeteil gewechselt werden.

Grundsätzlich wäre es denkbar, daß Längsachsen der Schraubenaufnahmen quer zur Drehachse angeordnet sind. Allerdings läßt sich der Schraubenbehälter wesentlich kompakter gestalten, wenn in jeder der Mehrzahl von Entnahmestellungen jeweils nur eine Entnahmeöffnung mit einer der Schraubenaufnahmen koaxial ausrichtbar ist. Insbesondere bei parallel zur Drehachse ausgerichteten Schraubenaufnahmen können diese alle parallel zu ihren Längsachsen im Schraubenbehälter aufbewahrt und auch parallel zu ihren Achsen eingeführt und/oder entnommen werden.

Vorteilhaft ist es, wenn ein Positioniermechanismus umfassend eine Mehrzahl von Positioniergliedern vorgesehen ist zum definierten Positionieren des Verschlußteils in mindestens einer Verschlußstellung und/oder in mindestens einer Entnahmestellung, daß mindestens eines der Positionierglieder am Aufnahmeteil und mindestens ein zu diesem korrespondierendes Positionierglied am Verschlußteil vorgesehen ist und daß mindestens zwei zueinander korrespondierende Positionierglieder am Verschlußteil und am Aufnahmeteil ineinandergreifen, wenn in der mindestens einen Entnahmestellung mindestens eine Entnahmeöffnung des Verschlußteils eine einzige Schraubenaufnahme freigibt. Durch die Positioniervorrichtung kann so auf einfache Weise sichergestellt werden, daß das Verschlußteil relativ zum Aufnahmeteil in definierter Weise eine Entnahmestellung und/oder eine Verschlußstellung einnimmt. Beispielsweise kann der Positioniermechanismus in Form eines Rastmechanismus ausgebildet sein, so daß eine Bedienperson das Einnehmen einer Verschlußstellung und/oder einer Entnahmestellung sowohl spüren als auch akustisch wahrnehmen kann.

Vorzugsweise umfassen die Positionierglieder Positioniervorsprünge und Positionierausnehmungen. Auf diese Weise läßt sich der Aufbau des Schraubenkörpers weiter vereinfachen.

Ein besonders kompakter Aufbau des Schraubenbehälters wird ermöglicht, wenn die Positionierglieder konzentrisch um die Drehachse angeordnet sind. Beispielsweise könnten die Schraubenaufnahmen selbst als Positionierglied dienen, in die ein korrespondierender Vorsprung eintaucht.

Damit der Schraubenbehälter möglichst viele Schrauben oder Werkzeugeinsätze aufnehmen kann, ist es günstig, wenn die Positionierglieder in gleichen Winkelabständen um die Drehachse herum angeordnet sind. Man könnte auch sagen, daß die Positionierglieder in Umfangsrichtung gleichmäßig verteilt angeordnet sind. Auf diese Weise läßt sich zudem auch ein hochsymmetrischer Aufbau des Schraubenbehälters realisieren.

Gemäß einer bevorzugten Ausführungsform der Erfindung kann ferner vorgesehen sein, daß das Aufnahmeteil einen in Richtung auf das Verschlußteil weisenden ringförmigen Rand aufweist und daß der Rand die am Aufnahmeteil vorgesehenen Positionierglieder trägt. Der Rand kann zum einen als Anschlag für das Verschlußteil dienen und zum anderen die Positionierglieder tragen. Besonders leicht lassen sich die Positionierglieder des Verschlußteils mit denen des Aufnahmeteils in Eingriff bringen, wenn an einer in Richtung auf das Aufnahmeteil weisenden Unterseite des Verschlußteils mindestens ein Positionierglied angeordnet ist. Beispielsweise kann mit einem derart angeordneten Positionierglied ein Positioniermechanismus mit den auf einem Rand des Aufnahmeteils angeordneten Positioniergliedern ausgebildet werden.

Vorteilhaft ist es, wenn die Anzahl der Positionieraufnahmen einer ganzzahligen Potenz der Zahl 2 entspricht, wenn höchstens so viele Positioniervorsprünge wie Positionieraufnahmen vorgesehen sind und wenn der Winkelabstand der Positionieraufnahmen dem Quotienten von 360° und der Anzahl der Positionieraufnahmen entspricht. Beispielsweise können vier, acht, sechzehn oder zweiunddreißig Positionieraufnahmen und maximal ebenso viele Positioniervorsprünge vorgesehen sein, wobei dann ein Winkelabstand der Positionieraufnahmen 90°, 45°, 22,5° bzw. 11,25° beträgt.

Um den Aufbau des chirurgischen Schraubenbehälters so einfach wie möglich zu halten und trotzdem möglichst viele Schrauben oder Instrumenteneinsätze im Behälter aufbewahren zu können, ist es günstig, wenn mindestens zwei Entnahmeöffnungen vorgesehen sind, die auf mindestens zwei Radialstrahlen ausgehend von der Drehachse angeordnet sind, und wenn die mindestens zwei Radialstrahlen zwischen sich einen Drehwinkel einschließen. Durch entsprechende Wahl des Drehwinkels bzw. durch entsprechende Anordnung der Schraubenaufnahmen kann sichergestellt werden, daß pro Entnahmestellung nur eine Schraubenaufnahme frei zugänglich ist zum Einführen und/oder Entnehmen einer Schraube.

Vorteilhafterweise entspricht der Drehwinkel einem ganzzahligen Vielfachen des Winkelabstands der Positionierglieder am Verschlußteil und/oder am Aufnahmeteil. Damit ist es möglich, beispielsweise in jeder oder in jeder zweiten Winkelstellung des Verschlußteils relativ zum Aufnahmeteil die an beiden Teilen angeordneten Positionierglieder in Eingriff zu bringen, so daß definiert Entnahmestellungen und/oder Verschlußstellungen eingenommen werden.

Günstig ist es, wenn mindestens zwei Entnahmeöffnungen, die unterschiedliche Abstände von der Drehachse aufweisen, vorgesehen sind und wenn der von den Radialstrahlen, auf denen die Entnahmeöffnungen angeordnet sind, eingeschlossene Drehwinkel 45° beträgt. Sind mehr als zwei auf mehr als zwei Radialstrahlen angeordnete Entnahmeöffnungen vorgesehen, so kann der eingeschlossene Drehwinkel auch kleiner als 45° sein. Vorzugsweise entspricht der eingeschlossene Drehwinkel dem doppelten Winkelabstand zweier Positionierglieder.

Besonders einfach wird der Aufbau des Schraubenbehälters, wenn das mindestens eine Magazinkupplungsglied ein Rastelement umfaßt zum in Eingriff Bringen mit einem korrespondieren Rastelement des Schraubenbehältermagazins.

Damit der Schraubenbehälter auch in unterschiedlichen Positionen mit einem Schraubenbehältermagazin lösbar verbindbar ist, ist es günstig, wenn mehrere gleichmäßig über den Umfang des Schraubenbehälters verteilte Magazinkupplungsglieder vorgesehen sind. Beispielsweise kann ein insgesamt eine vierzählige Symmetrie aufweisender Schraubenbehälter mit vier in einem Winkelabstand von 90° über seinen Umfang verteilte Magazinkupplungsgliedern ausgestattet sein.

Vorteilhaft ist es, wenn der Schraubenbehälter mindestens zwei in entgegengesetzten Richtungen abstehende Kupplungsvorsprünge trägt, die korrespondierend zu zwei parallelen, aufeinander zu weisenden Nuten von mindestens einer Schraubenbehälteraufnahme eines chirurgischen Schraubenbehältermagazins, welches mindestens einen chirurgischen Schraubenbehälter aufnehmen kann, ausgebildet und in die Nuten einführbar sind. So wird erreicht, daß der Schraubenbehälter am Schraubenbehältermagazin sicher gehalten werden kann. Durch die vorgesehenen Nuten wird eine Bewegung des Schraubenbehälters relativ zum Schraubenbehältermagazin auf einen Bewegungsfreiheitsgrad eingeschränkt.

Der Aufbau des Schraubenbehälters wird besonders einfach, wenn die mindestens zwei Kupplungsvorsprünge durch in einer Ebene liegende Lappen gebildet werden, die in Umfangsrichtung voneinander beabstandet sind. Beispielsweise können vier Lappen vorgesehen sein, welche zudem kleeblattförmig ausgebildet und angeordnet sein können.

Um den mit den Kupplungsvorsprüngen am Schraubenbehältermagazin gesicherten Schraubenbehälter zumindest temporär unbeweglich am Schraubenbehältermagazin festzulegen, ist es günstig, wenn jeweils zwischen zwei Kupplungsvorsprüngen ein Magazinkupplungsglied des Schraubenbehälters angeordnet ist. Dies ermöglicht es, den Schraubenbehälter in die Schraubenbehälteraufnahme in unterschiedlichen Stellungen einzuführen und auch in unterschiedlichen Stellungen mit dem Schraubenbehältermagazin lösbar zu verbinden.

Vier verschiedene Verbindungsstellungen zwischen dem Schraubenbehälter und einem Schraubenbehältermagazin sind möglich, wenn vier Kupplungsvorsprünge und vier Magazinkupplungsglieder vorgesehen sind und wenn eine Außenkontur der Kupplungsvorsprünge konzentrisch zur Drehachse ausgebildet ist.

Um eine Verdrehung des Schraubenbehälters in Nuten des Schraubenbehältermagazins zu verhindern, ist es günstig, wenn ein Abstand einer an zwei beabstandeten Kupplungsvorsprüngen anliegenden Tangente von der Drehachse kleiner ist als ein Abstand der konzentrischen Außenkontur der Kupplungsvorsprünge von der Drehachse. Dies bedeutet, daß durch den Abstand der Kupplungsvorsprünge voneinander quasi eine Sekante der ansonsten insgesamt eine scheibenförmige Außenkontur definierenden Kupplungsvorsprünge ausgebildet wird, die ein Verdrehen des Schraubenbehälters in Nuten des Schraubenbehältermagazins dann verhindern, wenn ein Abstand von Nutböden der Nuten zwar größer als der doppelte Abstand der Tangente von der Drehachse, jedoch kleiner als ein Außendurchmesser der konzentrischen Außenkontur der Kupplungsvorsprünge ist.

Günstigerweise weist das Verschlußteil Spülöffnungen auf. Dies ermöglicht es, den Schraubenbehälter nach einem chirurgischen Eingriff wieder aufzubereiten, ohne daß die nicht verwendeten Schrauben aus dem Schraubenbehälter entnommen werden müssen.

Um die Herstellung des Verschlußteils zu vereinfachen, sind die Spülöffnungen an der Deckplatte vorgesehen.

Damit die Reinigung sowohl des Schraubenbehälters als auch von darin aufbewahrten Schrauben oder Instrumenteneinsätzen verbessert wird, ist es vorteilhaft, wenn das Aufnahmeteil einen eine Längsrichtung definierenden hülsenförmigen Grundkörper und mindestens einen, den Grundkörper quer zur Längsrichtung durchsetzenden, die mindestens zwei Schraubenaufnahmen tragenden Steg umfaßt. Insbesondere kann der hülsenförmige Grundkörper in Form eines hohlzylindrischen Rings ausgebildet sein, der einen oder zwei, eine Längsachse des Grundkörpers kreuzende Stege aufweist.

Günstig ist es, wenn mindestens zwei sich kreuzende Stege vorgesehen sind und wenn die Zapfenaufnahme im Schnittpunkt der beiden Stege angeordnet ist. Dadurch wird zum einen die Stabilität des Schraubenbehälters erhöht, zum anderen wird auf einfache Weise eine Möglichkeit geschaffen, das Aufnahmeteil mit dem Verschlußteil zu verbinden, insbesondere durch Vorsehen eines zur Zapfenaufnahme korrespondierenden Kupplungszapfens am Verschlußteil.

Damit möglichst ein maximales Volumen des Schraubenbehälters zur Aufnahme von Schrauben oder Instrumenteneinsätzen genutzt werden kann, stehen vorzugsweise die Kupplungsvorsprünge flanschartig vom hülsenförmigen Grundkörper radial nach außen ab.

Um sowohl den Aufbau des Schraubenbehälters als auch einen Aufbau eines Schraubenbehältermagazins möglichst kompakt zu halten, ist es vorteilhaft, wenn die Magazinkupplungsglieder flanschartig vom hülsenförmigen Grundkörper radial nach außen abstehen.

Grundsätzlich wäre es denkbar, das Verschlußteil relativ zum Aufnahmeteil von Hand zu bewegen. Um jedoch bei einem chirurgischen Eingriff möglichst eine Verletzung von von Chirurgen getragenen chirurgischen Handschuhen zu vermeiden, ist es günstig, wenn das Verschlußteil mindestens ein Werkzeugkupplungsglied für ein Betätigungswerkzeug zum Verdrehen des Verschlußteils relativ zum Aufnahmeteil trägt. Ist beispielsweise das Aufnahmeteil mit Kupplungsvorsprüngen und eventuell zusätzlich vorhandenen Magazinkupplungsgliedern in einer Schraubenbehälteraufnahme eines Schraubenbehältermagazins eingeführt, dann kann durch das Betätigungswerkzeug das Verschlußteil auf einfache Weise relativ zum Aufnahmeteil verdreht werden.

Der Aufbau des Schraubenbehälters wird besonders einfach, wenn das mindestens eine Werkzeugkupplungsglied ein von der Deckplatte des Verschlußteils abstehender Vorsprung ist. Ein solcher Vorsprung dient alternativ auch als Angriffspunkt, um das Verschlußteil relativ zum Aufnahmeteil von Hand verdrehen zu können.

Damit sichergestellt werden kann, daß das mindestens eine Werkzeugkupplungsglied definiert mit einem Betätigungswerkzeug in Eingriff gebracht werden kann, ist es günstig, wenn zwei, bezogen auf die Drehachse diametral gegenüberliegende Werkzeugkupplungsglieder vorgesehen sind. Dadurch kann ein Drehmoment symmetrisch auf das Verschlußteil übertragen werden.

Um den Schraubenbehälter zusammen mit darin enthaltenen Schrauben aufzubereiten, insbesondere sterilisieren zu können, ist es günstig, wenn der Schraubenbehälter aus einem sterilisierbaren Kunststoff hergestellt ist.

Damit man sofort erkennen kann, wie viele Schraubenaufnahmen des Schraubenbehälters befüllt sind und um welche es sich dabei handelt, ist es vorteilhaft, wenn das Verschlußteil aus einem transparenten Material hergestellt ist.

Günstigerweise ist in jeder Schraubenaufnahme eine Knochenschraube angeordnet. Der Schraubenbehälter umfaßt also zusätzlich auch die in den Schraubenaufnahmen enthaltenen Knochenschrauben oder Werkzeugeinsätze.

Die eingangs gestellte Aufgabe wird ferner gelöst durch ein chirurgisches Schraubenbehältermagazin für mindestens einen der oben beschriebenen chirurgischen Schraubenbehälter, und zwar dadurch, daß das Schraubenbehältermagazin mindestens eine Schraubenbehälteraufnahme ausweist, in welche der mindestens eine Schraubenbehälter mindestens teilweise einführbar ist. Dies ermöglicht es, ein oder mehrere Schraubenbehälter zusammenzufassen, beispielsweise Schraubenbehälter, die mit unterschiedlich großen Schrauben bestückt sind. Dadurch können für einen chirurgischen Eingriff Schrauben unterschiedlicher Größe bereitgestellt werden, wobei es die oben beschriebenen vorteilhaften Ausgestaltungen des Schraubenbehälters gestatten, jeweils nur eine einzige Schraube oder einen Instrumenteneinsatz aus dem Schraubenbehälter zu entnehmen. Dies bedeutet aber auch, daß aus dem gesamten Schraubenbehältermagazin mit mehreren Schraubenbehältern jeweils nur eine einzelne Schraube oder ein Instrumenteneinsatz entnehmbar ist. Ferner hat dies den Vorteil, daß eine bereits entnommene Schraube oder ein Instrumenteneinsatz wieder in den dafür vorgesehenen Schraubenbehälter zurückgebracht werden kann, und zwar nachdem der gewünschte Schraubenbehälter in die erforderliche Entnahmestellung für die zur Aufnahme der Schraube vorgesehene Schraubenaufnahme gebracht wird.

Besonders einfach lassen sich Schraubenbehälter mit dem Schraubenbehältermagazin verbinden, wenn der mindestens eine Schraubenbehälter in die Schraubenbehälteraufnahme einschiebbar ist.

Günstigerweise umfaßt das chirurgische Schraubenbehältermagazin mindestens einen der oben beschriebenen erfindungsgemäßen Schraubenbehälter. Das Schraubenbehältermagazin und der mindestens eine Schraubenbehälter bilden somit eine Einheit. Beispielsweise kann das Schraubenbehältermagazin so bereits mit einem oder mehreren Schraubenbehältern zum Einsatz in der Chirurgie angeboten werden.

Vorteilhaft ist es, wenn der mindestens eine chirurgische Schraubenbehälter mit dem Schraubenbehältermagazin rastend verbindbar ist. Dies vereinfacht sowohl den Aufbau des Schraubenbehälters als auch den des Schraubenbehältermagazins. Insbesondere ist es denkbar, beide Teile aus einem Kunststoff in Spritzgußtechnik herzustellen.

Um sicherzustellen, daß sich ein oder mehrere Schraubenbehälter nicht in unerwünschter Weise vom Schraubenbehältermagazin lösen können, ist es günstig, wenn der mindestens eine chirurgische Schraubenbehälter mit dem Schraubenbehältermagazin in einer Lagerstellung, in welcher der mindestens eine Schraubenbehälter vollständig in die Schraubenbehälteraufnahme eingeführt ist, mit dem Schraubenbehältermagazin verbunden ist. Insbesondere kann der Schraubenbehälter auch wieder vom Schraubenbehältermagazin lösbar sein, um ihn aus der Lagerstellung herauszubewegen.

Damit auf einfache Weise der Schraubenbehälter und das Schraubenbehältermagazin in der Lagerstellung verbunden werden können, ist es vorteilhaft, wenn der mindestens eine Schraubenbehälter und das Schraubenbehältermagazin jeweils ein Magazinkupplungsglied tragen, die in der Lagerstellung in Eingriff stehen. Eine Verbindung zwischen dem Schraubenbehälter und dem Schraubenbehältermagazin ergibt sich somit in der Lagerstellung, nämlich dann, wenn die jeweiligen Magazinkupplungsglieder in Eingriff stehen.

Um auf einfache Weise eine lösbare Verbindung zwischen dem Schraubenbehälter und dem Schraubenbehältermagazin zu erhalten, ist es günstig, wenn eines der beiden Magazinkupplungsglieder ein Rastelement in Form einer federnd gelagerten Rastnase und wenn das andere Magazinkupplungsglied ein Rastelement in Form eines mit der Rastnase zusammenwirkenden Rücksprungs ausgebildet ist. Beispielsweise kann der Rücksprung auch in Form einer Durchbrechung ausgebildet sein, in die die Rastnase in der Lagerstellung ganz oder teilweise eintauchen kann.

Damit Schraubenbehälter in jeder Schraubenbehälteraufnahme sicher in der Lagerstellung gehalten sind, ist vorteilhafterweise jeder Schraubenbehälteraufnahme ein Magazinkupplungsglied des Schraubenbehältermagazins zugeordnet.

Einfach und sicher lassen sich Schraubenbehälter mit dem Schraubenbehältermagazin verbinden, wenn die Schraubenbehälteraufnahmen Kupplungsaufnahmen in Form von jeweils zwei parallelen aufeinander zu weisenden Nuten umfassen, in welche korrespondierende Kupplungsvorsprünge des mindestens einen Schraubenbehälters einführbar sind. Denkbar wäre es auch, daß die parallelen Nuten voneinander weg weisen, so daß korrespondierende Magazinkupplungsglieder des mindestens einen Schraubenbehälters die Schraubenbehälteraufnahme mindestens teilweise umgreifen.

Die eingangs gestellte Aufgabe wird ferner durch eine chirurgische Bereitstellungsbox für chirurgische Implantate und/oder chirurgische Werkzeuge, mit mehreren Abteilen zur Aufnahme chirurgischer Implantate und/oder Instrumente und/oder chirurgischer Werkzeuge erfindungsgemäß dadurch gelöst, daß mindestens ein Abteil zur Aufnahme eines der oben beschriebenen, erfindungsgemäßen Schraubenbehältermagazine vorgesehen ist. Das erfindungsgemäße Schraubenbehältermagazin bildet somit einen Teil einer chirurgischen Bereitstellungsbox, in welcher ein Teil oder sämtliche der für einen chirurgischen Eingriff benötigten Instrumente, Werkzeuge und/oder Implantate enthalten sind. Dies ermöglichst es, zur Vorbereitung eines bestimmten chirurgischen Eingriffs, alle erforderlichen Teile in die chirurgische Bereitstellungsbox einzulegen. Diese ist vorzugsweise sterilisierbar, so daß die vorbereitete und bestückte Bereitstellungsbox vor dem Eingriff sterilisiert werden kann.

Damit der Schraubenbehälter von der mindestens einen Verschlußstellung in mindestens eine Entnahmestellung bringbar ist, ist es günstig, wenn die Bereitstellungsbox mindestens eine Aufnahme für einen Schraubendreher und/oder einen Schraubendrehergriff aufweist. Mit diesen Werkzeugen läßt sich zum einen eine Knochenschraube, beispielsweise in einen Knochen, einschrauben, zum anderen kann mit einem entsprechend ausgestalteten Griffende das Verschlußteil relativ zum Aufnahmeteil bewegt werden.

Vorteilhafterweise umfaßt die Bereitstellungsbox mindestens einen Schraubendreher und/oder einen Schraubendrehergriff, welcher ein proximales Ende aufweist, welches eine Werkzeugaufnahme trägt zum in Eingriff Bringen mit einem Werkzeugkupplungsglied des Verschlußteils eines der oben beschriebenen Schraubenbehälter. Mit dem Schraubendreher bzw. dem Schraubendrehergriff kann auf einfache Weise das Verschlußteil relativ zum Aufnahmeteil bewegt werden, um das Verschlußteil von der mindestens einen Verschlußstellung in die mindestens eine Entnahmestellung überzuführen und eine Schraube oder einen Instrumenteneinsatz aus dem Schraubenbehälter zu entnehmen.

Vorzugsweise umfaßt die Werkzeugaufnahme mindestens zwei diametral gegenüberliegende, in proximaler Richtung weisende Ausnehmungen. Mit einer solchen Werkzeugaufnahme ist es möglich, zwei, bezogen auf die Drehachse des Schraubenbehälters diametral gegenüberliegende Werkzeugkupplungsglieder aufzunehmen und das Verschlußteil relativ zum Aufnahmeteil zu verdrehen.

Um die gewünschten Instrumente, Werkzeuge und/oder Implantate in geordnetem Zustand für einen chirurgischen Eingriff bereitstellen und sie leicht entnehmen zu können, ist es vorteilhaft, wenn mindestens ein Teil der Abteile im wesentlichen korrespondierend zu einer äußeren Kontur der darin gelagerten Implantate, Instrumente, Werkzeuge oder Schraubenbehältermagazine ausgebildet ist.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht einer bestückten chirurgischen Bereitstellungsbox;
- Figur 2:: eine Draufsicht auf ein Schraubenbehältermagazin mit einem Schraubenbehälter in der Lagerstellung;
- Figur 3:: eine Schnittansicht längs Linie 3-3 in Figur 2;
- Figur 4:: eine Seitenansicht des Schraubenbehältermagazins in Figur 2 in Richtung des Pfeils A;
- Figur 5:: eine vergrößerte Ansicht des Ausschnitts B in Figur 2;
- Figur 6:: eine Draufsicht auf ein Aufnahmeteil eines Schraubenbehälters;
- Figur 7:: eine Ansicht eines Verschlußteils eines Schraubenbehälters von unten;
- Figur 8a:: eine Schnittansicht längs Linie 8a-8a in Figur 6;
- Figur 8b:: eine Schnittansicht längs Linie 8b-8b in Figur 7; und
- Figur 9:: eine perspektivische Ansicht eines Schraubenbehälters mit einem Schraubendrehergriff.

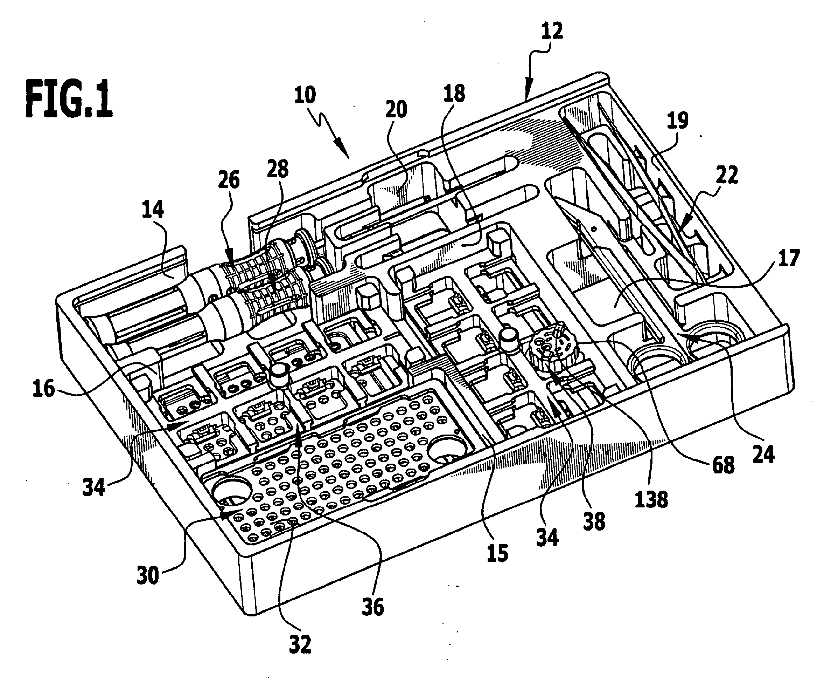

In Figur 1 ist eine insgesamt mit dem Bezugszeichen 10 versehene chirurgische Bereitstellungsbox dargestellt, welche eine im wesentlichen quaderförmige Behälterwanne 12 umfaßt, die mehrere unterschiedlich geformte Abteile aufweist, welche in Figur 1 beispielhaft mit den Bezugszeichen 14, 15, 16, 17, 18, 19 und 20 versehen sind.

Die beispielhaft genannten Abteile 14 bis 20 sind derart geformt, daß chirurgische Instrumente, beispielsweise eine insgesamt mit dem Bezugszeichen 22 versehene Pinzette oder eine insgesamt mit dem Bezugszeichen 24 versehene Schere, im jeweiligen Abteil, beispielsweise die Pinzette 22 im Abteil 19 oder die Schere 24 im Abteil 17, gelagert und auf einfache Weise entnommen werden können. Ferner sind im Abteil 14 ein Schraubendreher 26 sowie ein Schraubendrehergriff 28 angeordnet, welcher mit unterschiedlichen Schraubeinsätzen für Knochenschrauben bedarfsweise bestückt werden kann. Ferner umfaßt die Bereitstellungsbox 10 verschiedene Magazine, beispielsweise ein im Abteil 15 angeordnetes Kleinteilemagazin 30 mit einem gelochten Schiebedeckel 32. Die Abteile 16 und 18 dienen zur Aufnahme jeweils eines Schraubenbehältermagazins 34, die jeweils 8 im wesentlichen identisch ausgebildete Schraubenbehälteraufnahmen 36 zur Aufnahme jeweils eines dosenförmigen Schraubenbehälters 38 aufweisen.

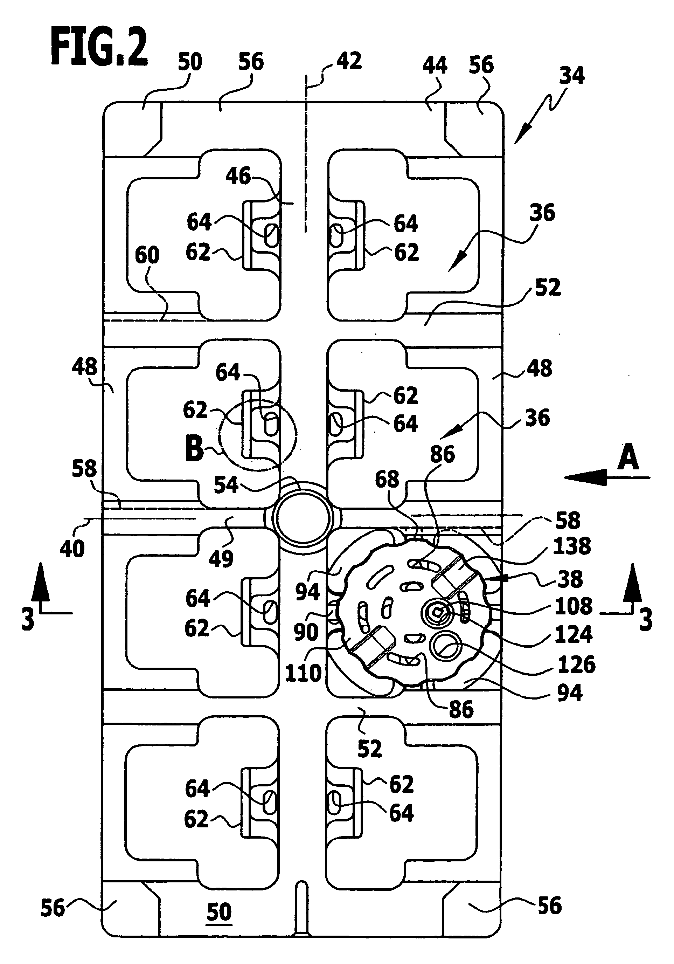

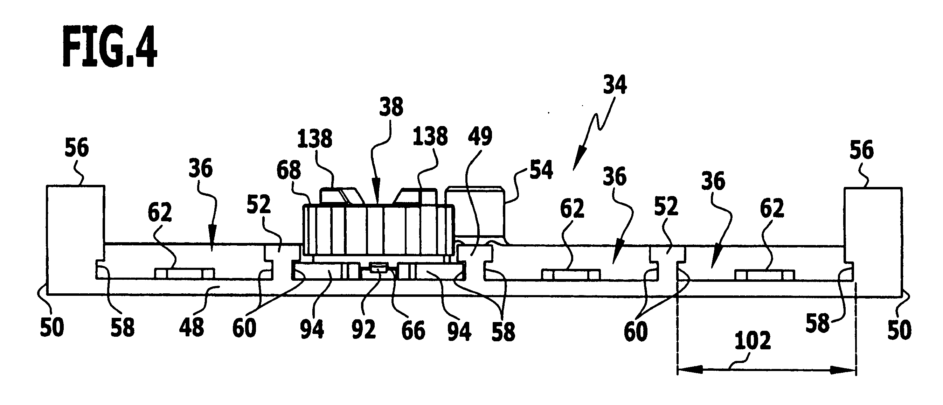

Das nachfolgend in Verbindung mit den Figuren 2 bis 4 näher beschriebene Schraubenbehältermagazin 34 ist spiegelsymmetrisch zu zwei einander senkrecht schneidenden Symmetrieebenen 40 und 42 ausgebildet. Es umfaßt einen langgestreckten rechteckigen Rahmen 44 mit einem zentralen, die Symmetrieebene 42 enthaltenden Längssteg 46, symmetrisch zu diesem angeordnete Seitenstege 48 sowie einen die Symmetrieebene 40 enthaltenden Mittelsteg 49. Symmetrisch zum Mittelsteg 49 angeordnete äußere Querstege 50 und zwischen den Mittelsteg 49 und den Querstegen 50 angeordnete, parallel zu diesen verlaufende Trennstege 58. Im Schnittpunkt des Längsstegs 46 und des Mittelstegs 49 steht in Richtung einer Schnittlinie der Symmetrieebenen 40 und 42 nach oben ein Haltezapfen 54 ab. Ebenfalls nach oben stehen in den Ecken des Rahmens 44, also in den Schnittpunkten der Seitenstege 48 und der Querstege 50, im wesentlichen quaderförmige Abstandshalter 56 ab.

Die Schraubenbehälteraufnahmen 36 werden jeweils begrenzt durch einen Trennsteg 52, den Längssteg 46, einen Seitensteg 48 und entweder den Mittelsteg 49 oder einen der Querstege 50. Jede Schraubenbehälteraufnahme 36 umfaßt zwei parallele Nuten 58 und 60, deren Nutböden aufeinander zu weisen und parallel zur Symmetrieebene 40 ausgebildet sind. Nutseitenwände der Nuten 58 und 60 definieren Ebenen, die sowohl zur Symmetrieebene 40 als auch zur Symmetrieebene 42 senkrecht orientiert sind. Die Nuten 58 sind jeweils in den Querstegen 50 sowie im Mittelsteg 49 angeordnet, die Nuten 60 im Mittelsteg 49. Die Nuten 58 und 60 erstrecken sich jeweils über etwa zwei Drittel der freien Länge der Stege, in denen sie angeordnet sind, ausgehend von einer äußeren Kante der Seitenstege 48.

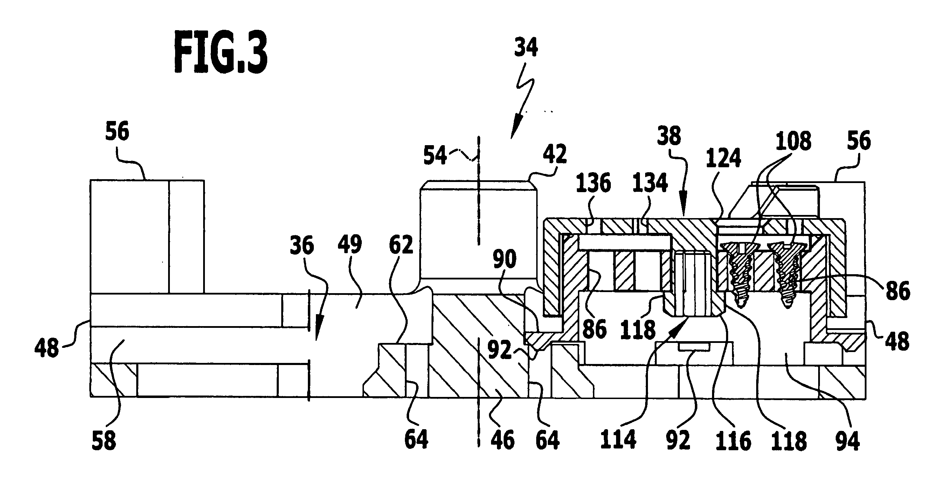

Beidseits des Längsstegs 46 stehen von diesem in Richtung auf die Seitenstege 48 weisend im wesentlichen quaderförmige Vorsprünge 62 ab, die von Langlöchern 64 in einer Richtung parallel zur Schnittlinie der Symmetrieebene 40 und 42 durchsetzt werden. Die Langlöcher 64 bilden rücksprungartige Magazinkupplungsglieder zum lösbaren Verbinden des Schraubenbehältermagazins mit einem Schraubenbehälter 38, wie dies in Figur 3 dargestellt und nachfolgend im Zusammenhang mit der näheren Beschreibung des Schraubenbehälters 38 erläutert ist.

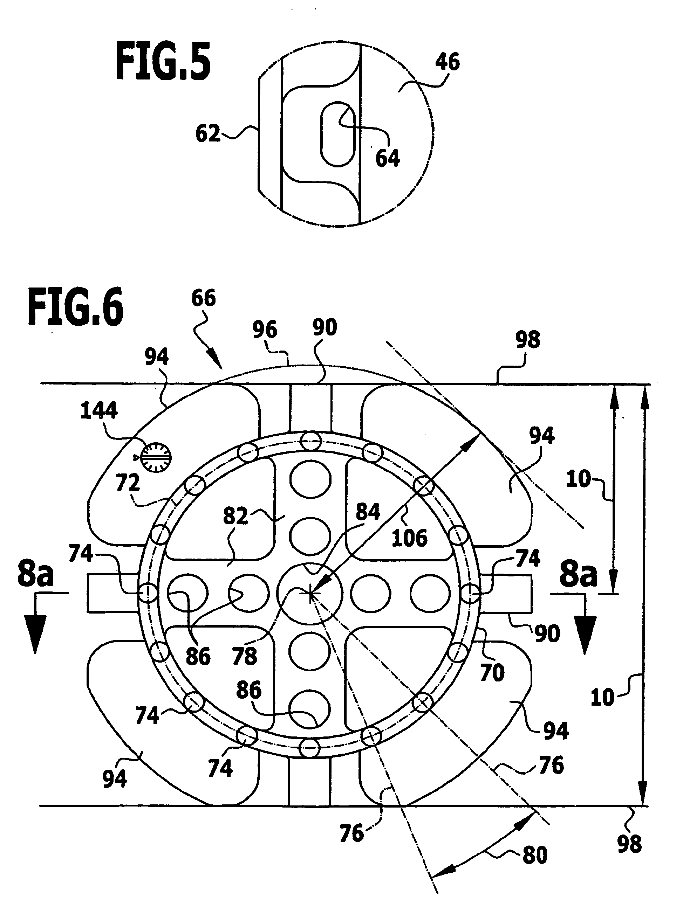

Der in den Figuren 1 bis 4 und 9 dargestellte Schraubenbehälter 38 wird nachfolgend im Zusammenhang mit den Figuren 6 bis 8b im einzelnen beschrieben.

Der Schraubenbehälter 38 ist zweiteilig ausgebildet und umfaßt ein mit dem Bezugszeichen 66 versehenes Aufnahmeteil sowie einen ein Verschlußteil bildenden Deckel 68. Das Aufnahmeteil 66 umfaßt einen Grundkörper 70 in Form eines zylindrischen Hülsenabschnitts mit einem nach oben weisenden Stirnrand 72, welcher 16 gleichmäßig über seinen Umfang verteilte Vertiefungen 74 aufweist, die Positionierglieder des Aufnahmeteils 66 bilden. Sie sind jeweils auf Radialstrahlen 76 ausgehend von einer Längsachse 78 des Grundkörpers angeordnet und um einen Winkel 80 versetzt, der sich berechnet aus dem Quotienten aus 360° und der Anzahl der Vertiefungen 74 und somit 22,5° beträgt. Quer zur Längsachse 78 durchsetzen den Grundkörper 70 zwei Stege 82, die sich in der Längsachse 78 schneiden. Im Schnittpunkt der Stege 82 und konzentrisch zur Längsachse 78 ist eine Zapfenaufnahme bildende Bohrung 84 angeordnet. Des weiteren sind auf den Stegen zwischen der Bohrung 84 und dem Grundkörper 70 jeweils zwei Schraubenaufnahmen 86 bildende Bohrungen vorgesehen, deren Längsachse parallel zur Längsachse 78 verlaufen und deren Durchmesser etwas kleiner sind als der der Bohrung 84.

Von einem unteren Rand 88 des Aufnahmeteils 66 steht in Verlängerung der Stege 82 vom Grundkörper 70 radial nach außen jeweils ein Rastvorsprung 90 ab, der etwa halb so breit ist wie die Stege 82. Die Rastvorsprünge 90 tragen auf ihren Unterseiten Rastnasen 92, die von oben her kommend in die Langlöcher 64 eintauchen können. Der Rastvorsprung 90 mit seiner Rastnase 92 bildet ein Magazinkupplungsglied des Schraubenbehälters 38.

Zwischen zwei Rastvorsprüngen 90 erstreckt sich, beabstandet von diesen, jeweils ein radial nach außen abstehender, eine kreisbogenförmige Außenkontur 96 aufweisender Kupplungsvorsprung 94. Die Außenkontur 96 ist in Figur 6 gestrichelt angedeutet. Ferner sind die Kupplungsvorsprünge 94 so konstruiert, daß eine an die Rastvorsprünge 90 angelegte Tangente 98 gleichzeitig auch eine Begrenzungslinie für die Kupplungsvorsprünge 94 bildet. Ein Abstand 100 zwischen jeweils zwei parallelen Tangenten 98 entspricht einem Abstand 102 von aufeinander zu weisenden Nutböden von einander zugeordneten Nuten 58 und 60 einer Schraubenbehälteraufnahme 36. Damit ist jedoch auch ein Abstand 104 der Tangente 98 von der Längsachse 78 kleiner als ein Radius 106, der durch die Außenkontur 96 definiert wird. Dies ermöglicht es, den Schraubenbehälter 38 parallel zu einem seiner Stege 82 in eine Schraubenbehälteraufnahme 36 einzuführen, wobei jeweils in entgegengesetzter Richtung voneinander weg weisende Rastvorsprünge 90 sowie an Tangenten 98 anliegende Bereiche der Kupplungsvorsprünge 94 in die Nuten 58 bzw. 60 eintauchen und in diesen geführt werden. Damit läßt sich der Schraubenbehälter 38 in Richtung auf den Längssteg 46 verschieben, bis er seine Lagerstellung einnimmt, in welcher die am Vorsprung 62 beim Einschieben aufgleitende Rastnase 92 durch Rückfedern des Rastvorsprungs 90 in das Langloch 64 eintaucht, wie beispielsweise in Figur 3 gut zu erkennen ist.

Die Schraubenaufnahmen 86 dienen zur Aufnahme von Knochenschrauben 108, von denen zwei beispielhaft in Figur 3 dargestellt sind. Ein Außendurchmesser eines Schraubenkopfs der Knochenschraube 108 ist etwas größer als ein Durchmesser der die Schraubenaufnahmen 86 bildenden Bohrungen, so daß ein Schraubenkörper der Knochenschraube 108 zwar in die Schraubenaufnahme eingeführt werden kann, ein Rand derselben jedoch einen Anschlag für den Kopf der Knochenschraube 108 bildet.

Damit in die Schraubenaufnahmen 86 eingesetzte Knochenschrauben 108 nicht in unbeabsichtigter Weise aus den Schraubenaufnahmen 86 herausfallen können, ist der Deckel 68 vorgesehen. Er umfaßt eine scheibenförmige Deckplatte 110 sowie eine von dieser abstehende hohlzylindrische Ringwand 112, deren Innendurchmesser etwas größer ist als ein Außendurchmesser des Grundkörpers 70. Dadurch kann der Deckel 68 mit der Ringwand 112 voran von oben über das Aufnahmeteil 66 geschoben werden.

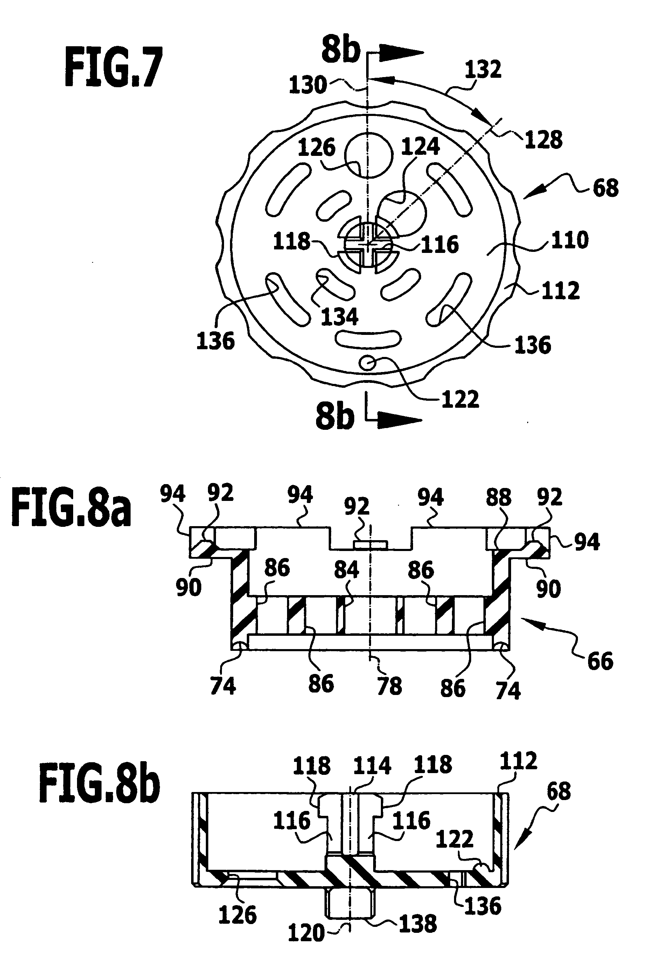

Zum Verbinden des Deckels 68 mit dem Aufnahmeteil 66 dient ein auf einer Unterseite der Deckplatte 110 abstehender Kupplungszapfen 114, der durch vier Rastvorsprünge 116 gebildet wird, die an ihren freien Enden radial nach außen vorspringende Rastnasen 118 tragen. Der Kupplungszapfen 114 kann mit den Rastnasen 118 voran in die Bohrung 84 eingeführt werden, wodurch die Rastvorsprünge 116 in Richtung auf eine Längsachse des Kupplungszapfens 114 verschwenkt werden und erst dann wieder in ihre in Figur 8b dargestellte Grundstellung zurückfedern, wenn die Rastnasen 118 aus der Bohrung 84 vorstehen und diese seitlich hintergreifen, wie in Figur 3 zu erkennen ist. Durch die Ausgestaltung von Deckel 68 und Aufnahmeteil 66 ist es möglich, diese beiden Teile relativ zueinander um die Längsachsen 78 bzw. 120 zu verdrehen.

Definierte Drehstellungen des Deckels 68 relativ zum Aufnahmeteil 66 lassen sich einstellen, wenn ein halbkugelförmiges, von einer Unterseite der Deckplatte 110 abstehendes Positionierglied 122 in eine der sechzehn Vertiefungen 74 eintaucht. Es lassen sich so sechzehn definierte Winkelstellungen des Deckels 68 relativ zum Aufnahmeteil realisieren. Wird der Deckel aus einer definierten Winkelstellung herausbewegt, so gleitet das Positionierglied 122 auf dem Stirnrand 72 auf und führt zu einer temporären Verformung des Deckels 68, bis es in eine benachbarte Vertiefung 74 eintauchen kann. Das Positionierglied 122 in Verbindung mit den Vertiefungen 74 bildet somit einen Positioniermechanismus.

Der Deckel 68 ist ferner mit zwei durch Bohrungen gebildete Entnahmeöffnungen 124 und 126 versehen, die jeweils auf einem Radialstrahl 128 bzw. 130 angeordnet sind, die zwischen sich einen Winkel 132 von 45° einschließen. Die Entnahmeöffnung 124 ist in einem Abstand von der Längsachse 120 angeordnet, so daß sie konzentrisch zur Deckung mit den Schraubenaufnahmen 86 gebracht werden kann, die benachbart der Bohrung 84 angeordnet sind. Der Abstand der Entnahmeöffnung 126 von der Längsachse 120 ist so gewählt, daß die Entnahmeöffnung 126 konzentrisch zur Deckung gebracht werden kann mit den Schraubenaufnahmen 86, die benachbart zum Grundkörper 70 angeordnet sind.

Der Deckel 68 weist ferner langlochartige Spülöffnungen 134 und 136 auf, wobei drei Spülöffnungen 134 konzentrisch zur Längsachse 120 angeordnet sind und eine Breite in radialer Richtung aufweisen, die kleiner ist als ein Innendurchmesser der Schraubenaufnahmen 86, und deren Abstand von der Längsachse 120 in etwa dem Abstand des Zentrums der Entnahmeöffnung 124 von der Längsachse 120 entspricht. In ähnlicher Weise sind fünf langlochartige Spülöffnungen 136 die Längsachse 120 konzentrisch umgebend vorgesehen, deren Abstand von der Längsachse 120 etwa dem Abstand des Zentrums der Entnahmeöffnung 126 von der Längsachse 120 entspricht.

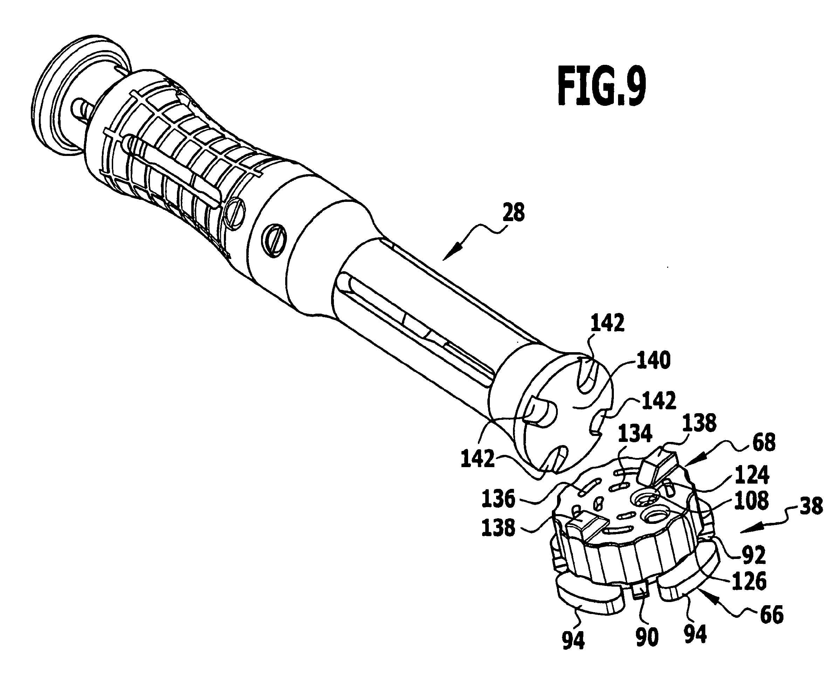

Auf einer Außenseite des Deckels 68 sind zwei einander diametral gegenüberliegende, in radialer Richtung weisende Werkzeugkupplungsglieder 138 angeordnet. Sie dienen als Verdrehhilfe, um den Deckel 68 relativ zum Aufnahmeteil 66 zu verdrehen, beispielsweise per Hand oder mit einem Schraubendreher 26 oder einem Schraubendrehergriff 28. In Figur 9 ist ein Schraubendrehergriff 28 dargestellt, welcher an seinem proximalen Ende 140 in proximaler Richtung weisende Werkzeugaufnahmen 142 aufweist, in die jeweils ein Werkzeugkupplungsglied 138 eintauchen kann. Der Schraubendrehergriff 28 kann also mit seinem proximalen Ende auf den Deckel 68 aufgesteckt werden und durch Rotation um seine Längsachse kann der Deckel 68 relativ zum Aufnahmeteil 66 verdreht werden, wenn das Aufnahmeteil 66 festgehalten wird, beispielsweise wenn es sich in einer Schraubenbehälteraufnahme 36 in der Lagerstellung befindet.

Durch die besondere Ausgestaltung des Schraubenbehälters 38 wird sichergestellt, daß nur jeweils eine der beiden Entnahmeöffnungen 124 und 126 mit genau einer der Schraubenaufnahmen 86 in Deckung gebracht werden kann, so daß diese zum Einführen oder Entnehmen einer Knochenschraube 108 zugänglich wird. Dies ist möglich, da die beiden Entnahmeöffnungen 124 und 126 um den Winkel 132 in Umfangsrichtung versetzt angeordnet sind, die Schraubenaufnahmen, die einen unterschiedlichen Abstand von der Längsachse 78 haben, dagegen auf gleichen Radialstrahlen angeordnet sind. Selbstverständlich könnten im umgekehrten Fall auch die Entnahmeöffnungen auf demselben Radialstrahl angeordnet sein und Schraubenaufnahmen 86 mit unterschiedlichem Abstand von der Längsachse 78 auf um einen Winkel 132 versetzten Radialstrahlen.

Durch den oben beschriebenen Positioniermechanismus ist es möglich, den Deckel 68 relativ zum Aufnahmeteil 66 in sechzehn definierte Positionen zu bringen, wobei acht dieser Positionen sogenannte Entnahmestellungen definieren, in denen eine der beiden Entnahmeöffnungen 124 bzw. 126 jeweils genau eine der acht Schraubenaufnahmen freigibt. Die anderen acht definierten Positionen bilden sogenannte Verschlußstellungen, in denen die Entnahmeöffnungen 124 und 125 jeweils nur teilweise mit einer der Schraubenaufnahmen 86 überlappen, so daß zwar eine Durchtrittsöffnung verbleibt, die jedoch so klein ist, daß ein Kopf der Knochenschraube 108 nicht durch sie hindurchtreten kann. Dadurch sind die Knochenschrauben 108 in den acht Verschlußstellungen sicher im Schraubenbehälter 38 gehalten, insbesondere auch dann, wenn dieser auf den Kopf gestellt wird.

Der in Figur 9 dargestellte Schraubenbehälter 38 nimmt eine Entnahmestellung ein, in der die Entnahmeöffnung 124 eine Schraubenaufnahme 86 freigibt. Wird der Deckel 68 relativ zum Aufnahmeteil 66 um 22,5° verdreht, dann taucht das Positionierglied 122 wieder in eine Vertiefung 74 ein, allerdings überlappt dann keine der beiden Entnahmeöffnungen 124 bzw. 126 vollständig mit einer Schraubenaufnahme. Dadurch nimmt der Schraubenbehälter 38 eine Verschlußstellung ein. Wird der Deckel 68 in der gleichen Richtung wiederum um weitere 22,5° verdreht, dann überlappt die Entnahmeöffnung 126 mit einer Schraubenaufnahme 86. Ein weiteres Verdrehen um 22,5° überführt den Schraubenbehälter 38 von einer Entnahmestellung wieder in eine Verschlußstellung. Durch diesen besonderen Positioniermechanismus werden bei Drehung des Deckels 68 abwechselnd Entnahmestellungen und Verschlußstellungen durchlaufen, wobei jeweils nach einer Verdrehung um 90° im Ergebnis identische Positionen eingenommen werden, beispielsweise ergibt sich dann wiederum eine wie in Figur 9 dargestellte Stellung des Schraubenbehälters 38.

Der Deckel 68 ist vorzugsweise aus einem transparenten Material hergestellt, so daß direkt erkennbar ist, in welcher der Schraubenaufnahmen 86 Knochenschrauben 108 enthalten sind. Zur Identifizierung der im Schraubenbehälter 38 enthaltenen Knochenschrauben 108 dient ein auf einem Kupplungsvorsprung 94 angeordneter Schraubencode 144, der beispielsweise durch Laserbeschriftung hergestellt ist.

Die zahlreiche Durchbrechungen umfassende Konstruktion des Schraubenbehälters 38 stellt sicher, daß in diesem enthaltene Knochenschrauben 108 mit dem Schraubenbehälter 38 sterilisiert werden können. Dies ist insbesondere auch dann möglich, wenn der Schraubenbehälter 38 im Schraubenbehältermagazin 34 angeordnet ist oder dieses wiederum in der Bereitstellungsbox 10 enthalten ist.

Die besondere Ausgestaltung des Schraubenbehälters 38 ermöglicht es, gezielt einzelne Knochenschrauben 108 zu entnehmen und, falls sie nicht benötigt werden, wieder in den Schraubenbehälter 38 einzuführen. Da der Schraubenbehälter 38 sterilisierbar ist, können nicht benötigte Knochenschrauben 108 mit dem Schraubenbehälter 38 nach einem erfolgten Eingriff wieder sterilisiert und für einen nächsten Eingriff bereitgestellt werden. Gegebenenfalls kann der Schraubenbehälter 38 auch mit einzelnen Knochenschrauben 108 wieder aufgefüllt werden.

## Patentansprüche

1. Chirurgischer Schraubenbehälter (38) umfassend einen Aufnahmeteil (66) und ein an diesem beweglich gelagertes Verschlußteil (68), wobei der Aufnahmeteil (66) mindestens zwei Schraubenaufnahmen (86) zum Aufnehmen von mindestens zwei Knochenschrauben (108) aufweist, wobei das Verschlußteil (68) relativ zum Aufnahmeteil (66) in mindestens eine Verschlußstellung bringbar ist, in welcher keine der Schraubenaufnahmen (86) zugänglich ist zum Einführen oder Entnehmen einer Knochenschraube (108), wobei das Verschlußteil (68) von der mindestens einen Verschlußstellung in mindestens zwei Entnahmestellungen bringbar ist, in welchen jeweils nur eine einzige Schraubenaufnahme (86) zugänglich ist zum Einführen oder Entnehmen einer einzelnen Knochenschraube (108), **dadurch gekennzeichnet, daß** am Schraubenbehälter (38) mindestens ein Magazinkupplungsglied (90, 92) vorgesehen ist zum lösbaren Verbinden des Schraubenbehälters (38) mit einem chirurgischen Schraubenbehältermagazin (34) für mindestens einen chirurgischen Schraubenbehälter (38).

2. Chirurgischer Schraubenbehälter nach Anspruch 1, **dadurch gekennzeichnet, daß** das Verschlußteil (68) am Aufnahmeteil (66) um eine Drehachse (78) verdrehbar gelagert ist.

3. Chirurgischer Schraubenbehälter nach Anspruch 2, **dadurch gekennzeichnet, daß** die mindestens zwei Schraubenaufnahmen (86) auf mindestens zwei konzentrischen Kreisen um die Drehachse (78) herum angeordnet sind.

4. Chirurgischer Schraubenbehälter nach einem der voranstehenden Ansprüche, **dadurch gekennzeichnet, daß** das Verschlußteil (68) zum beweglichen Lagern am Aufnahmeteil (66) mit diesem verrastbar ist.

5. Chirurgischer Schraubenbehälter nach einem der voranstehenden Ansprüche, **dadurch gekennzeichnet, daß** das Aufnahmeteil (66) und das Verschlußteil (68) zum beweglichen Lagern aneinander jeweils ein Kupplungselement (84, 114) tragen, die miteinander in Eingriff bringbar sind.

6. Chirurgischer Schraubenbehälter nach Anspruch 5, **dadurch gekennzeichnet, daß** die beiden Kupplungselemente (84, 114) in einer Kupplungsstellung relativ zueinander verdrehbar und mittels einer Rastverbindung aneinander gehalten sind.

7. Chirurgischer Schraubenbehälter nach einem der Ansprüche 5 oder 6, **dadurch gekennzeichnet, daß** das eine der beiden Kupplungselemente (84, 114) ein Kupplungszapfen (114) und daß das andere Kupplungselement (84) eine zum Kupplungszapfen korrespondierende Zapfenaufnahme (84) ist.

8. Chirurgischer Schraubenbehälter nach Anspruch 7, **dadurch gekennzeichnet, daß** der Kupplungszapfen (114) die Drehachse (78) definiert.

9. Chirurgischer Schraubenbehälter nach einem der voranstehenden Ansprüche, **dadurch gekennzeichnet, daß** das Verschlußteil (68) mindestens eine Entnahmeöffnung (124, 126) aufweist und daß die mindestens eine Entnahmeöffnung (124, 126) mindestens in einer der mindestens zwei Entnahmestellungen jeweils eine einzige Schraubenaufnahme (86) freigibt.

10. Chirurgischer Schraubenbehälter nach Anspruch 9, **dadurch gekennzeichnet, daß** das Verschlußteil (68) eine Deckplatte (110) zum mindestens teilweisen Bedecken der Schraubenaufnahmen (86) aufweist und daß die mindestens eine Entnahmeöffnung (124, 126) in Form einer Durchbrechung (124, 126) der Deckplatte (110) ausgebildet ist.

11. Chirurgischer Schraubenbehälter nach einem der Ansprüche 9 oder 10, **dadurch gekennzeichnet, daß** die mindestens eine Entnahmeöffnung (124, 126) konzentrisch um die Drehachse (78) angeordnet ist.

12. Chirurgischer Schraubenbehälter nach einem der Ansprüche 9 bis 11, **dadurch gekennzeichnet, daß** in jeder der Mehrzahl von Entnahmestellungen jeweils nur eine Entnahmeöffnung (124, 126) mit einer der Schraubenaufnahmen (86) koaxial ausrichtbar ist.

13. Chirurgischer Schraubenbehälter nach einem der voranstehenden Ansprüche, **dadurch gekennzeichnet, daß** ein Positioniermechanismus (74, 122) umfassend eine Mehrzahl von Positioniergliedern (74, 122) vorgesehen ist zum definierten Positionieren des Verschlußteils (68) in mindestens einer Verschlußstellung und/oder in mindestens einer Entnahmestellung, daß mindestens eines der Positionierglieder (74) am Aufnahmeteil (66) und mindestens ein zu diesem korrespondierendes Positionierglied (122) am Verschlußteil (68) vorgesehen ist und daß mindestens zwei zueinander korrespondierende Positionierglieder (74, 122) am Verschlußteil (68) und am Aufnahmeteil (66) ineinandergreifen, wenn in der mindestens einen Entnahmestellung mindestens eine Entnahmeöffnung (124, 126) des Verschlußteils (68) eine einzige Schraubenaufnahme (86) freigibt.

14. Chirurgischer Schraubenbehälter nach Anspruch 13, **dadurch gekennzeichnet, daß** die Positionierglieder (74, 122) konzentrisch um die Drehachse (78) angeordnet sind.

15. Chirurgischer Schraubenbehälter nach einem der Ansprüche 13 oder 14, **dadurch gekennzeichnet, daß** die Positionierglieder (74, 122) in gleichen Winkelabständen (80) um die Drehachse (78) herum angeordnet sind.

16. Chirurgischer Schraubenbehälter nach einem der Ansprüche 13 bis 15, **dadurch gekennzeichnet, daß** das Aufnahmeteil (66) einen in Richtung auf das Verschlußteil (68) weisenden ringförmigen Rand (72) aufweist und daß der Rand (72) die am Aufnahmeteil (66) vorgesehenen Positionierglieder (74) trägt.

17. Chirurgischer Schraubenbehälter nach einem der Ansprüche 13 bis 16, **dadurch gekennzeichnet, daß** an einer in Richtung auf das Aufnahmeteil (66) weisenden Unterseite des Verschlußteils (68) mindestens ein Positionierglied (122) angeordnet ist.

18. Chirurgischer Schraubenbehälter nach einem der Ansprüche 9 bis 17,
**dadurch gekennzeichnet, daß** mindestens zwei Entnahmeöffnungen (124, 126) vorgesehen sind, die auf mindestens zwei Radialstrahlen (128, 130) ausgehend von der Drehachse (78) angeordnet sind, und daß die mindestens zwei Radialstrahlen (128, 130) zwischen sich einen Drehwinkel (132) einschließen.

19. Chirurgischer Schraubenbehälter nach Anspruch 18, **dadurch gekennzeichnet, daß** der Drehwinkel (132) einem ganzzahligen Vielfachen des Winkelabstands (80) der Positionierglieder (74, 122) am Verschlußteil (68) und/oder am Aufnahmeteil (66) entspricht.

20. Chirurgischer Schraubenbehälter nach einem der Ansprüche 18 oder 19, **dadurch gekennzeichnet, daß** mindestens zwei Entnahmeöffnungen (124, 126), die unterschiedliche Abstände von der Drehachse (78) aufweisen, vorgesehen sind und daß der von den Radialstrahlen (128, 130), auf denen die Entnahmeöffnungen (124, 126) angeordnet sind, eingeschlossene Drehwinkel 45° beträgt.

21. Chirurgischer Schraubenbehälter nach einem der voranstehenden Ansprüche, **dadurch gekennzeichnet, daß** das mindestens eine Magazinkupplungsglied (90, 92) ein Rastelement (92) umfaßt zum in Eingriff Bringen mit einem korrespondierenden Rastelement (64) des Schraubenbehältermagazins (34).

22. Chirurgischer Schraubenbehälter nach einem der voranstehenden Ansprüche, **dadurch gekennzeichnet, daß** mehrere gleichmäßig über den Umfang des Schraubenbehälters (38) verteilte Magazinkupplungsglieder (90, 92) vorgesehen sind.

23. Chirurgischer Schraubenbehälter nach einem der voranstehenden Ansprüche, **dadurch gekennzeichnet, daß** der Schraubenbehälter (38) mindestens zwei in entgegengesetzten Richtungen abstehende Kupplungsvorsprünge (94) trägt, die korrespondierend zu zwei parallelen, aufeinander zu weisenden Nuten (58, 60) von mindestens einer Schraubenbehälteraufnahme (36) eines chirurgischen Schraubenbehältermagazins (34), welches mindestens einen chirurgischen Schraubenbehälter (38) aufnehmen kann, ausgebildet und in die Nuten (58, 60) einführbar sind.

24. Chirurgischer Schraubenbehälter nach Anspruch 23, **dadurch gekennzeichnet, daß** die mindestens zwei Kupplungsvorsprünge (94) durch in einer Ebene liegende Lappen (94) gebildet werden, die in Umfangsrichtung voneinander beabstandet sind.

25. Chirurgischer Schraubenbehälter nach einem der Ansprüche 23 oder 24, **dadurch gekennzeichnet, daß** jeweils zwischen zwei Kupplungsvorsprüngen (94) ein Magazinkupplungsglied (90, 92) des Schraubenbehälters (38) angeordnet ist.

26. Chirurgischer Schraubenbehälter nach einem der Ansprüche 23 bis 25, **dadurch gekennzeichnet, daß** vier Kupplungsvorsprünge (94) und vier Magazinkupplungsglieder (90, 92) vorgesehen sind und daß eine Außenkontur (96) der Kupplungsvorsprünge (94) konzentrisch zur Drehachse (78) ausgebildet ist.

27. Chirurgischer Schraubenbehälter nach einem der Ansprüche 23 bis 26, **dadurch gekennzeichnet, daß** ein Abstand (104) einer an zwei beabstandeten Kupplungsvorsprüngen (94) anliegenden Tangente (98) von der Drehachse (78) kleiner ist als ein Abstand (106) der konzentrischen Außenkontur (96) der Kupplungsvorsprünge (94) von der Drehachse (78).

28. Chirurgischer Schraubenbehälter nach einem der voranstehenden Ansprüche, **dadurch gekennzeichnet, daß** das Verschlußteil (68) Spülöffnungen (134, 136) aufweist.

29. Chirurgischer Schraubenbehälter nach einem der voranstehenden Ansprüche, **dadurch gekennzeichnet, daß** das Aufnahmeteil (66) einen eine Längsrichtung (78) definierenden hülsenförmigen Grundkörper (70) und mindestens einen, den Grundkörper (70) quer zur Längsrichtung durchsetzenden, die mindestens zwei Schraubenaufnahmen (86) tragenden Steg (82) umfaßt.

30. Chirurgischer Schraubenbehälter nach Anspruch 29, **dadurch gekennzeichnet, daß** die Magazinkupplungsglieder (90, 92) flanschartig vom hülsenförmigen Grundkörper (70) radial nach außen abstehen.

31. Chirurgischer Schraubenbehälter nach einem der voranstehenden Ansprüche, **dadurch gekennzeichnet, daß** das Verschlußteil (68) mindestens ein Werkzeugkupplungsglied (138) für ein Betätigungswerkzeug (26, 28) zum Verdrehen des Verschlußteils (68) relativ zum Aufnahmeteil (66) trägt.

32. Chirurgischer Schraubenbehälter nach Anspruch 31, **dadurch gekennzeichnet, daß** das mindestens eine Werkzeugkupplungsglied (138) ein von der Deckplatte (110) des Verschlußteils (68) abstehender Vorsprung (138) ist und/oder daß zwei, bezogen auf die Drehachse (78) diametral gegenüberliegende Werkzeugkupplungsglieder (138) vorgesehen sind.

33. Chirurgischer Schraubenbehälter nach einem der voranstehenden Ansprüche, **dadurch gekennzeichnet, daß** der Schraubenbehälter (38) aus einem sterilisierbaren Kunststoff hergestellt ist.

34. Chirurgischer Schraubenbehälter nach einem der voranstehenden Ansprüche, **dadurch gekennzeichnet, daß** das Verschlußteil (68) aus einem transparenten Material hergestellt ist.

35. Chirurgischer Schraubenbehälter nach einem der voranstehenden Ansprüche, **dadurch gekennzeichnet, daß** in jeder Schraubenaufnahme (86) eine Knochenschraube (108) angeordnet ist.

36. Chirurgisches Schraubenbehältermagazin (34) für mindestens einen chirurgischen Schraubenbehälter (38) nach einem der voranstehenden Ansprüche, **dadurch gekennzeichnet, daß** das Schraubenbehältermagazin (34) mindestens eine Schraubenbehälteraufnahme (36) aufweist, in welche der mindestens eine Schraubenbehälter (38) mindestens teilweise einführbar ist.

37. Chirurgisches Schraubenbehältermagazin nach Anspruch 36, **dadurch gekennzeichnet, daß** der mindestens eine Schraubenbehälter (38) in die Schraubenbehälteraufnahme (36) einschiebbar ist.

38. Chirurgisches Schraubenbehältermagazin nach einem der Ansprüche 36 oder 37, **dadurch gekennzeichnet, daß** das Schraubenbehältermagazin (34) mindestens einen Schraubenbehälter (38) nach einem der Ansprüche 1 bis 35 umfaßt.

39. Chirurgisches Schraubenbehältermagazin nach Anspruch 38, **dadurch gekennzeichnet, daß** der mindestens eine chirurgische Schraubenbehälter (38) mit dem Schraubenbehältermagazin (34) rastend verbindbar ist.

40. Chirurgisches Schraubenbehältermagazin nach Anspruch 39, **dadurch gekennzeichnet, daß** der mindestens eine chirurgische Schraubenbehälter (38) mit dem Schraubenbehältermagazin (34) in einer Lagerstellung, in welcher der mindestens eine Schraubenbehälter (38) vollständig in die Schraubenbehälteraufnahme (36) eingeführt ist, verbunden ist.

41. Chirurgisches Schraubenbehältermagazin nach Anspruch 40, **dadurch gekennzeichnet, daß** der mindestens eine Schraubenbehälter (38) und das Schraubenbehältermagazin (34) jeweils ein Magazinkupplungsglied tragen, die in der Lagerstellung in Eingriff stehen.

42. Chirurgisches Schraubenbehältermagazin nach Anspruch 41, **dadurch gekennzeichnet, daß** eines der beiden Magazinkupplungsglieder (64, 90, 92) ein Rastelement (92) in Form einer federnd gelagerten Rastnase (92) und daß das andere Magazinkupplungsglied (64) ein Rastelement (64) in Form eines mit der Rastnase zusammenwirkenden Rücksprungs (64) ausgebildet ist.

43. Chirurgisches Schraubenbehältermagazin nach einem der Ansprüche 41 oder 42, **dadurch gekennzeichnet, daß** jeder Schraubenbehälteraufnahme (36) ein Magazinkupplungsglied (64) des Schraubenbehältermagazins (34) zugeordnet ist.

44. Chirurgisches Schraubenbehältermagazin nach einem der Ansprüche 36 bis 43, **dadurch gekennzeichnet, daß** die Schraubenbehälteraufnahmen (36) Kupplungsaufnahmen (58, 60) in Form zweier paralleler, aufeinander zu weisender Nuten (58, 60) umfassen, in welche korrespondierende Kupplungsvorsprünge (94) des mindestens einen Schraubenbehälters (38) einführbar sind.

45. Chirurgische Bereitstellungsbox (10) für chirurgische Implantate und/oder chirurgische Werkzeuge (22, 24, 26, 28), mit mehreren Abteilen zur Aufnahme chirurgischer Implantate und/oder Instrumente (22, 24, 26, 28) und/oder chirurgischer Werkzeuge, **dadurch gekennzeichnet, daß** mindestens ein Abteil (16, 18) zur Aufnahme eines Schraubenbehältermagazins (34) nach einem der Ansprüche 36 bis 44 vorgesehen ist.

46. Chirurgische Bereitstellungsbox nach Anspruch 45, **dadurch gekennzeichnet, daß** die Bereitstellungsbox (10) mindestens eine Aufnahme für einen Schraubendreher (26) und/oder einen Schraubendrehergriff (28) aufweist.

47. Chirurgische Bereitstellungsbox nach einem der Ansprüche 45 oder 46, **dadurch gekennzeichnet, daß** die Bereitstellungsbox (10) mindestens einen Schraubendreher (26) und/oder einen Schraubendrehergriff (28) umfaßt, welcher ein proximales Ende (140) aufweist, und daß das proximale Ende (140) eine Werkzeugaufnahme (142) trägt zum in Eingriff Bringen mit einem Werkzeugkupplungsglied (138) des Verschlußteils (68) eines Schraubenbehälters (38) nach einem der Ansprüche 1 bis 35.

48. Chirurgische Bereitstellungsbox nach Anspruch 47, **dadurch gekennzeichnet, daß** die Werkzeugaufnahme (142) mindestens zwei diametral gegenüberliegende, in proximaler Richtung weisende Ausnehmungen (142) umfaßt.

## Claims

1. Surgical screw container (38) comprising a receiving part (66) and a closure part (68) mounted thereon so as to be movable, wherein the receiving part (66) has at least two screw receptacles (86) for accommodating at least two bone screws (108), wherein the closure part (68) is adapted to be brought into at least one closure position relative to the receiving part (66), none of the screw receptacles (86) being accessible for the insertion or removal of a bone screw (108) in said closure position, wherein the closure part (68) is adapted to be brought from the at least one closure position into at least two removal positions, only a single screw receptacle (86) being accessible for the insertion or removal of a single bone screw (108) in each of said removal positions, **characterized in that** at least one storage device coupling member (90, 92) is provided on the screw container (38) for the releasable connection of the screw container (38) to a surgical screw container storage device (34) for at least one surgical screw container (38).

2. Surgical screw container as defined in claim 1, **characterized in that** the closure part (68) is mounted on the receiving part (66) so as to be rotatable about an axis of rotation (78).

3. Surgical screw container as defined in claim 2, **characterized in that** the at least two screw receptacles (86) are arranged on at least two concentric circles around the axis of rotation (78).

4. Surgical screw container as defined in any one of the preceding claims, **characterized in that** the closure part (68) is adapted to be connected to the receiving part (66) in a snap-in manner so as to be movably mounted thereon.

5. Surgical screw container as defined in any one of the preceding claims, **characterized in that** the receiving part (66) and the closure part (68) each bear a coupling element (84, 114) for the movable mounting on one another, said coupling elements being adapted to be brought into engagement with one another.

6. Surgical screw container as defined in claim 5, **characterized in that** the two coupling elements (84, 114) are rotatable relative to one another in a coupling position and are held on one another by means of a snap-in connection.

7. Surgical screw container as defined in one of claims 5 or 6, **characterized in that** one of the two coupling elements (84, 114) is a coupling pin (114) and that the other coupling element (84) is a pin receptacle (84) corresponding to the coupling pin.

8. Surgical screw container as defined in claim 7, **characterized in that** the coupling pin (114) defines the axis of rotation (78).

9. Surgical screw container as defined in any one of the preceding claims, **characterized in that** the closure part (68) has at least one removal opening (124, 126) and that the at least one removal opening (124, 126) frees a single screw receptacle (84) at least in one of the at least two respective removal positions.

10. Surgical screw container as defined in claim 9, **characterized in that** the closure part (68) has a cover plate (110) for covering the screw receptacles (86) at least partially and that the at least one removal opening (124, 126) is designed in the form of a passage (124, 126) in the cover plate (110).

11. Surgical screw container as defined in any one of claims 9 or 10, **characterized in that** the at least one removal opening (124, 126) is arranged concentrically around the axis of rotation (78).

12. Surgical screw container as defined in any one of claims 9 to 11, **characterized in that** in each of the plurality of removal positions only one removal opening (124, 126) is adapted to be aligned coaxially with one of the respective screw receptacles (86).

13. Surgical screw container as defined in any one of the preceding claims, **characterized in that** a positioning mechanism (74, 122) comprising a plurality of positioning members (74, 122) is provided for the defined positioning of the closure part (68) in at least one closure position and/or in at least one removal position, that at least one of the positioning members (74) is provided on the receiving part (66) and at least one positioning member (122) corresponding to it is provided on the closure part (68) and that at least two positioning members (74, 122) on the closure part (68) and on the receiving part (66), said members corresponding to one another, engage in one another when at least one removal opening (124, 126) of the closure part (68) frees a single screw receptacle (86) in the at least one removal position.

14. Surgical screw container as defined in claim 13, **characterized in that** the positioning members (74, 122) are arranged concentrically around the axis of rotation (78).

15. Surgical screw container as defined in any one of claims 13 or 14, **characterized in that** the positioning members (74, 122) are arranged at equal angular distances (80) around the axis of rotation (78).

16. Surgical screw container as defined in any one of claims 13 to 15, **characterized in that** the receiving part (66) has an annular edge (72) pointing in the direction towards the closure part (68) and that the edge (72) bears the positioning members (74) provided on the receiving part (66).

17. Surgical screw container as defined in any one of claims 13 to 16, **characterized in that** at least one positioning member (122) is arranged on an underside of the closure part (68) pointing in the direction towards the receiving part (66).

18. Surgical screw container as defined in any one of claims 9 to 17, **characterized in that** at least two removal openings (124, 126) are provided, said openings being arranged on at least two radial rays (128, 130) proceeding from the axis of rotation (78) and that the at least two radial rays (128, 130) form an angle of rotation (132) between them.

19. Surgical screw container as defined in claim 18, **characterized in that** the angle of rotation (132) corresponds to an integral multiple of the angular distance (80) between the positioning members (74, 122) on the closure part (68) and/or on the receiving part (66).

20. Surgical screw container as defined in one of claims 18 or 19, **characterized in that** at least two removal openings (124, 126) at different distances from the axis of rotation (78) are provided and that the angle of rotation formed by the radial rays (128, 130) is 45°, the removal openings (124, 126) being arranged on said radial rays.

21. Surgical screw container as defined in any one of the preceding claims, **characterized in that** the at least one storage device coupling member (90, 92) comprises a snap-in element (92) for bringing into engagement with a corresponding snap-in element (64) of the screw container storage device (34).

22. Surgical screw container as defined in any one of the preceding claims, **characterized in that** several storage device coupling members (90, 92) are provided, said coupling members being distributed uniformly over the circumference of the screw container (38).

23. Surgical screw container as defined in any one of the preceding claims, **characterized in that** the screw container (38) bears at least two coupling projections (94) projecting in opposite directions, said projections being designed to correspond to two parallel grooves (58, 60) of at least one screw container receptacle (36) of a surgical screw container storage device (34) and being insertable into the grooves (58, 60), said grooves pointing towards one another and said screw container storage device being able to accommodate at least one surgical screw container (38).

24. Surgical screw container as defined in claim 23, **characterized in that** the at least two coupling projections (94) are formed by tongues (94) located in one plane and spaced from one another in circumferential direction.

25. Surgical screw container as defined in one of claims 23 or 24, **characterized in that** a storage device coupling member (90, 92) of the screw container (38) is arranged between two respective coupling projections (94).

26. Surgical screw container as defined in any one of claims 23 to 25, **characterized in that** four coupling projections (94) and four storage device coupling members (90, 92) are provided and that an outer contour (96) of the coupling projections (94) is designed to be concentric to the axis of rotation (78).

27. Surgical screw container as defined in any one of claims 23 to 26, **characterized in that** a distance (104) of a tangent (98) touching two spaced coupling projections (94) from the axis of rotation (78) is smaller than a distance (106) of the concentric outer contour (96) of the coupling projections (94) from the axis of rotation (78).

28. Surgical screw container as defined in any one of the preceding claims, **characterized in that** the closure part (68) has wash openings (134, 136).

29. Surgical screw container as defined in any one of the preceding claims, **characterized in that** the receiving part (66) comprises a sleeve-like basic member (70) defining a longitudinal direction (78) and at least one web (82) passing through the basic member (70) transversely to the longitudinal direction and bearing the at least two screw receptacles (86).

30. Surgical screw container as defined in claim 29, **characterized in that** the storage device coupling members (90, 92) project radially outwards from the sleeve-like basic member (70) in a flange-like manner.

31. Surgical screw container as defined in any one of the preceding claims, **characterized in that** the closure part (68) has at least one tool coupling member (138) for an actuating tool (26, 28) for turning the closure part (68) relative to the receiving part (66).

32. Surgical screw container as defined in claim 31, **characterized in that** the at least one tool coupling member (138) is a projection (138) projecting from the cover plate (110) of the closure part (68) and/or **in that** two tool coupling members (138) located diametrically opposite one another in relation to the axis of rotation (78) are provided.

33. Surgical screw container as defined in any one of the preceding claims, **characterized in that** the screw container (38) is produced from a sterilizable plastic.

34. Surgical screw container as defined in any one of the preceding claims, **characterized in that** the closure part (68) is produced from a transparent material.

35. Surgical screw container as defined in any one of the preceding claims, **characterized in that** a bone screw (108) is arranged in each screw receptacle (86).

36. Surgical screw container storage device (34) for at least one surgical screw container (38) as defined in any one of the preceding claims, **characterized in that** the screw container storage device (34) has at least one screw container receptacle (36), the at least one screw container (38) being insertable into said receptacle at least partially.

37. Surgical screw container storage device as defined in claim 36, **characterized in that** the at least one screw container (38) is adapted to be pushed into the screw container receptacle (36).

38. Surgical screw container storage device as defined in one of claims 36 or 37, **characterized in that** the screw container storage device (34) comprises at least one screw container (38) as defined in any one of claims 1 to 35.

39. Surgical screw container storage device as defined in claim 38, **characterized in that** the at least one surgical screw container (38) is connectable to the screw container storage device (34) in a snap-in manner.

40. Surgical screw container storage device as defined in claim 39, **characterized in that** the at least one surgical screw container (38) is connected to the screw container storage device (34) in a storage position, the at least one screw container (38) being introduced completely into the screw container receptacle (36) in said position.

41. Surgical screw container storage device as defined in claim 40, **characterized in that** the at least one screw container (38) and the screw container storage device (34) each bear a storage device coupling member, said coupling members being in engagement in the storage position.

42. Surgical screw container storage device as defined in claim 41, **characterized in that** one of the two storage device coupling members (64, 90, 92) is designed as a snap-in element (92) in the form of a flexibly mounted snap-in nose (92) and that the other storage device coupling member (62) is designed as a snap-in element (64) in the form of a recess (64) interacting with the snap-in nose.

43. Surgical screw container storage device as defined in one of claims 41 or 42, **characterized in that** a storage device coupling member (64) of the screw container storage device (34) is associated with each screw container receptacle (36).

44. Surgical screw container storage device as defined in any one of claims 36 to 43, **characterized in that** the screw container receptacles (36) comprise coupling receptacles (58, 60) in the form of two parallel grooves (58, 60) pointing towards one another, corresponding coupling projections (94) of the at least one screw container (38) being insertable into said grooves.

45. Surgical supply box (10) for surgical implants and/or surgical tools (22, 24, 26, 28), comprising several compartments for accommodating surgical implants and/or instruments (22, 24, 26, 28) and/or surgical tools, **characterized in that** at least one compartment (16, 18) is provided for accommodating a screw container storage device (34) as defined in any one of claims 36 to 44.

46. Surgical supply box as defined in claim 45, **characterized in that** the supply box (10) has at least one receptacle for a screwdriver (26) and/or a screwdriver handle (28).

47. Surgical supply box as defined in one of claims 45 or 46, **characterized in that** the supply box (10) comprises at least one screwdriver (26) and/or a screwdriver handle (28) having a proximal end (140) and that the proximal end (140) bears a tool receptacle (142) for bringing into engagement with a tool coupling member (138) of the closure part (68) of a screw container (38) as defined in any one of claims 1 to 35.

48. Surgical supply box as defined in claim 47, **characterized in that** the tool receptacle (142) comprises at least two recesses (142) located diametrically opposite one another and pointing in a proximal direction.

## Revendications

1. Boitier à vis chirurgical (38) comprenant une pièce de réception (66) et une pièce de fermeture (68) qui y est montée de manière mobile, boitier
dans lequel la pièce de réception (66) présente au moins deux emplacements de réception de vis (86) destinés à recevoir au moins deux vis à os (108),
dans lequel la pièce de fermeture (68) peut être amenée, par rapport à la pièce de réception (66), dans au moins une position de fermeture dans laquelle aucun des emplacements de réception de vis (86) n'est accessible pour l'introduction ou le prélèvement d'une vis à os (108), et
dans lequel la pièce de fermeture (68) peut être amenée de ladite au moins une position de fermeture à au moins deux positions de prélèvement, dans chacune desquelles un seul emplacement de réception de vis (86) est accessible pour l'introduction ou le prélèvement d'une vis à os individuelle (108),
**caractérisé en ce que** sur le boitier à vis (38) est prévu au moins un organe de couplage de magasin (90, 92) pour assurer la liaison amovible du boitier à vis (38) avec un magasin de boitiers à vis chirurgical (34) pour au moins un boitier à vis chirurgical (38).

2. Boitier à vis chirurgical selon revendication 1, **caractérisé en ce que** la pièce de fermeture (68) est montée sur la pièce de réception (66), de manière rotative autour d'un axe de rotation (78).

3. Boitier à vis chirurgical selon la revendication 2, **caractérisé en ce que** lesdits au moins deux emplacements de réception de vis (86) sont agencés sur au moins deux cercles concentriques autour de l'axe de rotation (78).

4. Boitier à vis chirurgical selon l'une des revendications précédentes, **caractérisé en ce que** la pièce de fermeture (68), pour le montage mobile sur la pièce de réception (66), peut être encliquetée avec celle-ci.

5. Boitier à vis chirurgical selon l'une des revendications précédentes, **caractérisé en ce que** la pièce de réception (66) et la pièce de fermeture (68), pour le montage mobile de l'une par rapport à l'autre, portent chacune un élément de couplage (84, 114), qui peuvent être amenés en prise réciproque.

6. Boitier à vis chirurgical selon la revendication 5, **caractérisé en ce que** les deux éléments de couplage (84, 114) sont, dans une position de couplage, rotatifs l'un par rapport à l'autre et maintenus l'un à l'autre au moyen d'une liaison par encliquetage.

7. Boitier à vis chirurgical selon l'une des revendications 5 ou 6, **caractérisé en ce que** l'un des deux éléments de couplage (84, 114) est un tourillon de couplage (114), et **en ce que** l'autre élément de couplage (84) est un logement de tourillon (84) correspondant au tourillon de couplage.

8. Boitier à vis chirurgical selon la revendication 7, **caractérisé en ce que** le tourillon de couplage (114) définit l'axe de rotation (78).

9. Boitier à vis chirurgical selon l'une des revendications précédentes, **caractérisé en ce que** la pièce de fermeture (68) présente au moins une ouverture de prélèvement (124, 126), et **en ce que** ladite au moins une ouverture de prélèvement (124, 126) dégage, dans au moins l'une desdites au moins deux positions de prélèvement, respectivement un seul emplacement de réception de vis (86).

10. Boitier à vis chirurgical selon la revendication 9, **caractérisé en ce que** la pièce de fermeture (68) présente une plaque de recouvrement (110) destinée à recouvrir au moins partiellement les emplacements de réception de vis (86), et **en ce que** ladite au moins une ouverture de prélèvement (124, 126) est réalisée sous la forme d'un passage traversant (124, 126) de la plaque de recouvrement (110).

11. Boitier à vis chirurgical selon l'une des revendications 9 ou 10, **caractérisé en ce que** ladite au moins une ouverture de prélèvement (124, 126) est agencée de manière concentrique autour de l'axe de rotation (78).

12. Boitier à vis chirurgical selon l'une des revendications 9 à 11, **caractérisé en ce que** dans chacune des plusieurs positions de prélèvement, seule à chaque fois une ouverture de prélèvement (124, 126) peut être orientée coaxialement avec l'un des emplacements de réception de vis (86).

13. Boitier à vis chirurgical selon l'une des revendications précédentes, **caractérisé en ce qu'**il est prévu un mécanisme de positionnement (74, 122) comprenant plusieurs organes de positionnement (74, 122) pour le positionnement défini de la pièce de fermeture (68) dans au moins une position de fermeture et/ou dans au moins une position de prélèvement, **en ce qu'**au moins l'un des organes de positionnement (74) est prévu sur la pièce de réception (66) au moins un organe de positionnement (122) correspondant au précédent est prévu sur la pièce de fermeture (68), et **en ce qu'**au moins deux organes de positionnement (74, 122) mutuellement correspondants sur la pièce de fermeture (68) et sur la pièce de réception (66), viennent s'engager l'un dans l'autre lorsque, dans ladite au moins une position de prélèvement, au moins une ouverture de prélèvement (124, 126) de la pièce de fermeture (68) dégage un seul emplacement de réception de vis (86).

14. Boitier à vis chirurgical selon la revendication 13, **caractérisé en ce que** les organes de positionnement (74, 122) sont agencés concentriquement autour de l'axe de rotation (78).

15. Boitier à vis chirurgical selon l'une des revendications 13 ou 14, **caractérisé en ce que** les organes de positionnement (74, 122) sont agencés selon des espacements angulaires (80) identiques autour de l'axe de rotation (78).

16. Boitier à vis chirurgical selon l'une des revendications 13 à 15, **caractérisé en ce que** la pièce de réception (66) présente un bord (72) de forme annulaire, qui est dirigé en direction de la pièce de fermeture (68), et **en ce que** le bord (72) porte les organes de positionnement (74) prévus sur la pièce de réception (66).

17. Boitier à vis chirurgical selon l'une des revendications 13 à 16, **caractérisé en ce que** sur une face inférieure de la pièce de fermeture (68) dirigée en direction de la pièce de réception (66), est agencé au moins un organe de positionnement (122).

18. Boitier à vis chirurgical selon l'une des revendications 9 à 17, **caractérisé en ce que** sont prévues au moins deux ouvertures de prélèvement (124, 126), qui sont agencées sur au moins deux rayons radiaux (128, 130) issus de l'axe de rotation (78), et **en ce que** lesdits au moins deux rayons radiaux (128, 130) forment entre eux un angle de rotation (132).

19. Boitier à vis chirurgical selon la revendication 18, **caractérisé en ce que** l'angle de rotation (132) correspond à un multiple entier de l'espacement angulaire (80) des organes de positionnement (74, 122) sur la pièce de fermeture (68) et/ou sur la pièce de réception (66).

20. Boitier à vis chirurgical selon l'une des revendications 18 ou 19, **caractérisé en ce que** sont prévues au moins deux ouvertures de prélèvement (124, 126) situées à des distances différentes de l'axe de rotation (78), et **en ce que** l'angle de rotation formé entre les rayons radiaux (128, 130) sur lesquels sont agencées les ouvertures de prélèvement (124, 126), vaut 45°.

21. Boitier à vis chirurgical selon l'une des revendications précédentes, **caractérisé en ce que** ledit au moins un organe de couplage de magasin (90, 92) comprend un élément d'encliquetage (92) destiné à être amené en prise avec un élément d'encliquetage correspondant (64) du magasin de boitiers à vis (34).

22. Boitier à vis chirurgical selon l'une des revendications précédentes, **caractérisé en ce que** sont prévus plusieurs organes de couplage de magasin (90, 92) répartis de manière régulière le long de la périphérie du boitier à vis (38).

23. Boitier à vis chirurgical selon l'une des revendications précédentes, **caractérisé en ce que** le boitier à vis (38) porte au moins deux protubérances de couplage (94) faisant saillie dans des directions opposées, qui sont réalisées en correspondance avec deux rainures (58, 60) parallèles, dirigées l'une vers l'autre, d'au moins un emplacement de réception de boitier à vis (36) d'un magasin de boitiers à vis chirurgical (34) pouvant recevoir au moins un boitier à vis chirurgical (38), et qui peuvent être introduites dans les rainures (58, 60).

24. Boitier à vis chirurgical selon la revendication 23, **caractérisé en ce que** lesdites au moins deux protubérances de couplage (94) sont formées par des pattes (94) situées dans un même plan, qui sont espacées mutuellement dans la direction périphérique.

25. Boitier à vis chirurgical selon l'une des revendications 23 ou 24, **caractérisé en ce qu'**entre à chaque fois deux protubérances de couplage (94), est agencé un organe de couplage de magasin (90, 92) du boitier à vis (38).

26. Boitier à vis chirurgical selon l'une des revendications 23 à 25, **caractérisé en ce que** sont prévus quatre protubérances de couplage (94) et quatre organes de couplage de magasin (90, 92), et **en ce qu'**un contour extérieur (96) des protubérances de couplage (94) est réalisé de manière à être concentrique à l'axe de rotation (78).

27. Boitier à vis chirurgical selon l'une des revendications 23 à 26, **caractérisé en ce qu'**une distance (104) d'une tangente (98) appliquée contre deux protubérances de couplage (94) espacées l'une de l'autre, à l'axe de rotation (78), est inférieure à une distance (106) du contour extérieur (96) concentrique des protubérances de couplage (94), à l'axe de rotation (78).

28. Boitier à vis chirurgical selon l'une des revendications précédentes, **caractérisé en ce que** la pièce de fermeture (68) présente des ouvertures de rinçage (134, 136).

29. Boitier à vis chirurgical selon l'une des revendications précédentes, **caractérisé en ce que** la pièce de réception (66) comprend un corps de base (70) en forme de douille définissant une direction longitudinale (78), et au moins une nervure (82) qui traverse le corps de base (70) transversalement à la direction longitudinale et porte lesdits au moins deux emplacements de réception de vis (86).

30. Boitier à vis chirurgical selon la revendication 29, **caractérisé en ce que** les organes de couplage de magasin (90, 92) font saillie radialement vers l'extérieur du corps de base (70) en forme de douille, à la manière d'un flasque.

31. Boitier à vis chirurgical selon l'une des revendications précédentes, **caractérisé en ce que** la pièce de fermeture (68) porte au moins un organe de couplage d'outil (138) pour un outil d'actionnement (26, 28) destiné à faire tourner la pièce de fermeture (68) par rapport à la pièce de réception (66).

32. Boitier à vis chirurgical selon la revendication 31, **caractérisé en ce que** ledit au moins un organe de couplage d'outil (138) est une protubérance (138) faisant saillie de la plaque de recouvrement (110) de la pièce de fermeture (68), et/ou **en ce que** sont prévus deux organes de couplage d'outil (138) diamétralement opposés par rapport à l'axe de rotation (78).

33. Boitier à vis chirurgical selon l'une des revendications précédentes, **caractérisé en ce que** le boitier à vis (38) est fabriqué en une matière plastique pouvant être stérilisée.

34. Boitier à vis chirurgical selon l'une des revendications précédentes, **caractérisé en ce que** la pièce de fermeture (68) est fabriquée en un matériau transparent.

35. Boitier à vis chirurgical selon l'une des revendications précédentes, **caractérisé en ce que** dans chaque emplacement de réception de vis (86) est agencée une vis à os (108).

36. Magasin de boitiers à vis chirurgical (34) destiné à au moins un boitier à vis chirurgical (38) selon l'une des revendications précédentes, **caractérisé en ce que** le magasin de boitiers à vis (34) présente au moins un emplacement de réception de boitier à vis (36) dans lequel peut être introduit au moins partiellement ledit au moins un boitier à vis (38).

37. Magasin de boitiers à vis chirurgical selon la revendication 36, **caractérisé en ce que** ledit au moins un boitier à vis (38) peut être inséré dans l'emplacement de réception de boitier à vis (36).

38. Magasin de boitiers à vis chirurgical selon l'une des revendications 36 ou 37, **caractérisé en ce que** le magasin de boitiers à vis (34) comprend au moins un boitier à vis (38) selon l'une des revendications 1 à 35.

39. Magasin de boitiers à vis chirurgical selon la revendication 38, **caractérisé en ce que** ledit au moins un boitier à vis chirurgical (38) peut être relié par encliquetage au magasin de boitiers à vis (34).

40. Magasin de boitiers à vis chirurgical selon la revendication 39, **caractérisé en ce que** ledit au moins un boitier à vis chirurgical (38) est relié au magasin de boitiers à vis (34) dans une position de stockage dans laquelle ledit au moins un boitier à vis (38) est inséré totalement dans l'emplacement de réception de boitier à vis (36).

41. Magasin de boitiers à vis chirurgical selon la revendication 40, **caractérisé en ce que** ledit au moins un boitier à vis (38) et le magasin de boitiers à vis (34) portent respectivement un organe de couplage de magasin, ceux-ci étant en prise réciproque dans la position de stockage.

42. Magasin de boitiers à vis chirurgical selon la revendication 41, **caractérisé en ce que** l'un des deux organes de couplage de magasin (64, 90, 92) est réalisé en tant qu'élément d'encliquetage (92) sous la forme d'un talon d'encliquetage (92) monté élastiquement, et **en ce que** l'autre organe de couplage de magasin (64) est réalisé en tant qu'élément d'encliquetage (64) sous la forme d'un cran de retenue (64) interagissant avec le talon d'encliquetage.

43. Magasin de boitiers à vis chirurgical selon l'une des revendications 41 ou 42, **caractérisé en ce qu'**à chaque emplacement de réception de boitier à vis (36) est associé un organe de couplage de magasin (64) du magasin de boitiers à vis (34).

44. Magasin de boitiers à vis chirurgical selon l'une des revendications 36 à 43, **caractérisé en ce que** les emplacements de réception de boitier à vis (36) comportent des logements de couplage (58, 60) sous la forme de deux rainures (58, 60) parallèles, dirigées l'une vers l'autre, dans lesquelles peuvent être introduites des protubérances de couplage (94) correspondantes dudit au moins un boitier à vis (38).

45. Coffret de présentation chirurgical (10) pour implants chirurgicaux et/ou outils chirurgicaux (22, 24, 26, 28), comprenant plusieurs compartiments destinés à recevoir des implants chirurgicaux et/ou des instruments (22, 24, 26, 28) et/ou des outils chirurgicaux, **caractérisé en ce qu'**il est prévu au moins un compartiment (16, 18) destiné à recevoir un magasin de boitiers à vis (34) selon l'une des revendications 36 à 44.

46. Coffret de présentation chirurgical selon la revendication 45, **caractérisé en ce que** le coffret de présentation (10) comporte au moins un emplacement de réception pour un tournevis (26) et/ou une poignée de tournevis (28).

47. Coffret de présentation chirurgical selon l'une des revendications 45 ou 46, **caractérisé en ce que** le coffret de présentation (10) comporte au moins un tournevis (26) et/ou une poignée de tournevis (28), qui présente une extrémité proximale (140), et **en ce que** l'extrémité proximale (140) porte un emplacement de réception d'outil (142) destiné à être amené en prise avec un organe de couplage d'outil (138) de la pièce de fermeture (68) d'un boitier à vis (38) selon l'une des revendications 1 à 35.

48. Coffret de présentation chirurgical selon la revendication 47, **caractérisé en ce que** l'emplacement de réception d'outil (142) comprend au moins deux évidements (142) diamétralement opposés, orientés en direction proximale.
